# EUROPEAN PATENT SPECIFICATION

(11) **EP 3 496 708 B1**
(45) Date of publication and mention of the grant of the patent: **26.03.2025**
(21) Application number: 17838221.4
(22) Date of filing: 10.08.2017
(51) Int. Cl.: A61K 31/19, A61K 9/00, A61P 37/00, A23L 33/12, A23L 33/00, A61K 9/16, A61P 37/06, A61K 47/50

(54) **METABOLITES FOR TREATMENT AND PREVENTION OF AUTOIMMUNE DISEASE**
METABOLITEN ZUR BEHANDLUNG UND PRÄVENTION VON AUTOIMMUNERKRANKUNGEN
MÉTABOLITES POUR LE TRAITEMENT ET LA PRÉVENTION DE MALADIE AUTO-IMMUNE

(30) Priority: 10.08.2016 AU 2016903143
(43) Date of publication of application: 19.06.2019
(73) Proprietor: Monash University, Melbourne, Victoria 3800 (AU); Commonwealth Scientific and Industrial Research Organisation, Acton, Australian Capital Territory 2601 (AU)
(72) Inventor: MACKAY, Charles Reay, Clayton Victoria 3800 (AU); MORENO, Eliana Marino, Clayton Victoria 3800 (AU); LOCKETT, Trevor, Acton Australian Capital Territory 2601 (AU); CLARKE, Julie, Acton Australian Capital Territory 2601 (AU); TOPPING, David, Acton Australian Capital Territory 2601 (AU)
(74) Representative: Kuttenkeuler, David
(86) International application number: PCT/AU2017/050845
(87) International publication number: WO 2018/027274

(56) References cited:
- WO-A1-01/02016
- WO-A1-2015/066433
- WO-A1-2015/066433
- WO-A1-2016/086210
- WO-A1-2016/086210
- US-A- 5 840 860
- US-A- 5 840 860
- DAVID L. TOPPING ET AL: "Resistant starches as a vehicle for delivering health benefits to the human large bowel", MICROBIAL ECOLOGY IN HEALTH AND DISEASE, vol. 20, no. 2, 1 January 2008 (2008-01-01), pages 103 - 108, XP055668747, DOI: 10.1080/08910600802106541
- ELIANA MARIÑO ET AL: "Gut microbial metabolites limit the frequency of autoimmune T cells and protect against type 1 diabetes", NATURE IMMUNOLOGY, vol. 18, no. 5, 27 March 2017 (2017-03-27), New York, pages 552 - 562, XP055463106, ISSN: 1529-2908, DOI: 10.1038/ni.3713
- DE GROOT PIETER F ET AL: "Oral butyrate does not affect innate immunity and islet autoimmunity in individuals with longstanding type 1 diabetes: a randomised controlled trial", DIABETOLOGIA, vol. 63, no. 3, 8 January 2020 (2020-01-08), pages 597 - 610, XP037331490, ISSN: 0012-186X, DOI: 10.1007/S00125-019-05073-8
- CLARKE J.M. ET AL.: "Acetylated High Amylose Maize Starch Improves the Efficacy of Oral Rehydration Solution in a Rat Model of Cholera", GASTROENTEROLOGY, vol. 140, no. 5, 2011, pages 134, XP055375908
- MARINO E ET AL.: "Gut microbial metabolites limit the frequency of autoimmune T cells and protect against type 1 diabetes", NATURE IMMUNOLOGY, vol. 18, no. 5, May 2017 (2017-05-01), pages 552 - 562, XP055463106
- BAJKA B.H. ET AL.: "Butyrylated starch is less susceptible to enzymic hydrolysis and increases large-bowel butyrate more than high-amylose maize starch in the rat", BRITISH JOURNAL OF NUTRITION, vol. 96, no. 2, 2006, pages 276 - 286, XP055463109

## Description

### Related application

This application claims priority from Australian provisional application AU 2016903143.

### Field of the invention

The invention relates to the combination and delivery of metabolite compounds for the treatment and prevention of autoimmune diseases.

### Background of the invention

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Autoimmune disease is a pathological state which arises from an abnormal immune response to organs and tissues in the body.

The burden of autoimmune disease is significant, with a substantial minority of the western population (2-5%) suffering from this group of diseases. Women are also more susceptible to autoimmune disease, particularly in child-bearing years, such that autoimmune disease is estimated as being among the leading causes of death of women in the US in all age groups up to 65.

There are no cures for autoimmune disease, and current therapies are typically aimed at managing the pain associated with the disease (for example, using steroids or non-steroidal anti-inflammatories) or at reducing the inflammatory response using immunosuppressants. Immunosuppressive pharmaceuticals can be prohibitively expensive, reducing access to therapy for many sufferers. In the case of autoimmune disease which results in the destruction of functional cells (for example, type 1 diabetes, in which pancreatic beta cells are destroyed), there are even more limited treatment options, with exogenous insulin treatment remaining the primary treatment approach.

WO2015/066433 A1 discloses methods, food supplements and pharmaceutical compositions for treating autoimmune diseases and inflammation based on the administration of activating agents able to increase the level of a short chain fatty acid to induce Treg differentiation in a subject, wherein the short chain fatty acid comprises butyrate, propionate or acetate covalently bonded to starch.

David L. Topping et al. (DOI: 10.1080/08910600802106541) discloses colonic delivery of acetylated, propionylated or butyrylated starches, wherein the bound SCFA is released by the large bowel microflora, for use in the treatment of inflammatory bowel disease.

WO2016/086210 A1 discloses the treatment of autoimmune disorders by administration of a microbial composition with anti-inflammatory bacteria into the gastrointestinal tract (which includes the large intestine) of the patient.

US 5 840 860 A discloses systems for delivery to the colon of short chain fatty acids bonded to starch and other suitable carriers.

WO 01/02016 A1 discloses nasogastric enteral formulations that comprise various components, including an amino acid source, a carbohydrate source, a lipid source and a fatty acid delivery agent. The compositions of are described as being useful for promoting bowel health.

Clarke J. M. et al. (DOI: 10.1016/S0016-5085(11)60547-7) discloses a whole-gut perfused rat model of cholera-toxin (CT) induced diarrhoea was to examine the effects of acylated starches in hypotonic oral rehydration solution on fluid and electrolyte uptake.

Bajka B.H. et al. (DOI: 10.1079/bjn20061807) has examined the effects of cooking of high-amylose maize starch and butyrylated high-amylose maize starch on amylolysis in vitro and their capacity to raise caeco-colonic short-chain fatty acids in rats.

A need exists for improved methods and compositions for treating and preventing autoimmune diseases.

The references to methods of treatment in the summary and detailed description of the invention in this description are to be interpreted as references to the compounds, pharmaceutical compositions and medicaments of the present invention for use in a method for treatment of the human (or animal) body by therapy.

### Summary of the invention

The present invention relates to a therapeutically effective amount of a combination of two or more short chain fatty acids, esters or salts thereof, for use in treating an autoimmune disease or for delaying or preventing the progression of an autoimmune disease in an individual, wherein the combination of short chain fatty acids is butyric acid and acetic acid, preferably wherein the combination further includes propionic acid, and wherein the short chain fatty acids are conjugated to a carrier in the form of a starch molecule.

In any embodiment of the invention, the individual may be determined to be at risk of developing an autoimmune disease. For example, the individual may have autoantibodies or inflammatory markers associated with a risk of developing an autoimmune disease.

Reducing or treating inflammation may include reducing the proportion of one or more pro-inflammatory cytokines in the individual. Further, reducing or treating inflammation may include increasing the proportion of one or more anti-inflammatory cytokines in the individual.

The present invention also provides a therapeutically effective amount of a combination of two or more short chain fatty acids, esters or salts thereof, for use in preventing or delaying the onset of autoimmune disease in an individual, wherein the combination of short chain fatty acids, esters or salts thereof, is butyric acid and acetic acid or esters or salts thereof, preferably wherein the combination further includes propionic acid, and wherein the short chain fatty acids are conjugated to a carrier in the form of a starch molecule.

The present invention also provides a dietary agent for delivery of a combination of short chain fatty acids into the large intestine of an individual, the agent including a carrier covalently bonded to the short chain fatty acids by a bond that is hydrolysable in the colon of an individual, to give free fatty acid, wherein the combination of short chain fatty acids is butyric acid and acetic acid, preferably wherein the combination further includes propionic acid, more preferably wherein the carrier is a carbohydrate selected from the group consisting of a starch, gum, oligosaccharide or pectin.

The present invention provides a pharmaceutical composition for use in treating or for use in delaying the progression of an autoimmune disease, wherein the composition includes a combination of butyric acid and acetic acid, esters or salts thereof and pharmaceutically acceptable excipients, wherein the butyric acid, acetic acid, esters or salts thereof are the active ingredients in the composition, and wherein the butyric acid and acetic acid are conjugated to a carrier in the form of a starch molecule.

Preventing, reducing or treating autoimmunity may include reducing the abundance or presence of one or more autoantibodies in the individual. The autoantibodies may be associated with a risk of autoimmune disease.

In any embodiment of the present invention, the autoimmune disease is selected from the group consisting of: type 1 diabetes, psoriasis, rheumatoid arthritis, inflammatory bowel disease, caeliac disease, autoimmune hepatitis, myocarditis, lupus nephritis, multiple sclerosis or primary biliary cirrhosis.

Preferably the autoimmune disease is type 1 diabetes.

In any embodiment of the present invention, the autoantibodies are associated with a risk of type 1 diabetes, including but not limited to islet autoantibodies, insulin autoantibodies and autoantibodies to glutamate decarboxylase (GAD) and islet-specific glucose-6-phosphatase catalytic subunit-related protein (IGPR).

In any embodiment of the present invention, the combination of two or more short chain fatty acids, esters or salts thereof, includes a combination of butyric acid and acetic acid, esters or salts thereof.

Preferably, the combinations, pharmaceutical composition, and dietary agent of the invention are for use in a method of treating or delaying the progression of type I diabetes in an individual, the method including providing in the individual, a therapeutically effective amount of butyric acid and acetic acid, esters or salts thereof, thereby treating or delaying the progression of type I diabetes.

Preferably, the combinations of the invention are for use in, a method of preventing or delaying the onset of type I diabetes in an individual, the method including providing in the individual, a therapeutically effective amount of butyric acid and acetic acid, esters or salts thereof, thereby preventing or delaying the onset of type I diabetes.

Preferably, the combinations, pharmaceutical composition, and dietary agent of the invention are for use in a method of treating or delaying the progression of type I diabetes in an individual, the method including providing in the large intestine of the individual, a therapeutically effective amount of butyric acid and acetic acid, esters or salts thereof, thereby treating or preventing the type I diabetes.

Preferably, the combinations are for use in a method of preventing or delaying the onset of type I diabetes in an individual, the method including providing in the large intestine of the individual, a therapeutically effective amount of butyric acid and acetic acid, esters or salts thereof, thereby preventing or delaying the onset of type I diabetes.

The combination may be butyric acid and acetic acid, or acetic acid, butyric acid and propionic acid.

As used herein, the terms acetate, butyrate, propionate and the like, refer to the salt form of the free acid, or, depending on the physiological environment, the free acid itself. In context, it may also refer to an ester of the free acid.

In any embodiment of the present invention, the combination of short chain fatty acids is provided in the large intestine of the individual. In any embodiment of the present invention, the combination of short chain fatty acids is provided in the colon of the individual. In any embodiment, the combination of short chain fatty acids is provided systemically in the individual (i.e., in the peripheral blood circulation). In any embodiment of the present invention, the combination of short chain fatty acids is provided in the individual by oral administration to the individual of a dietary agent or pharmaceutical composition including said short chain fatty acids. The dietary agent may include a carrier molecule covalently bonded to at least one short chain fatty acid, wherein the covalent bond is resistant to degradation in the small intestine of the individual but is hydrolysable in the colon to provide free fatty acid in the colon of the individual. Preferably, the carrier is a starch.

In any embodiment of the present invention, the administration of the combination of short chain fatty acids results in an increase in circulating levels of short chain fatty acids in the blood of the individual. In any embodiment of the invention, the increased circulating short chain fatty acid levels in the blood are sustained (i.e., not transient).

In any embodiment of the present invention, the administration of the combination of short chain fatty acids results in greater than, or equal to a 0.5-fold, 1-fold, 2-fold, 3-fold or 4-fold increase in the circulating levels of short chain fatty acids in the individual.

The present invention provides a pharmaceutical composition for use in treating or for use in delaying the progression of an autoimmune disease, wherein the composition includes a combination of butyric acid and acetic acid, esters or salts thereof and pharmaceutically acceptable excipients, wherein the butyric acid, acetic acid, esters or salts thereof are the active ingredients in the composition, and wherein the butyric acid and acetic acid are conjugated to a carrier in the form of a starch molecule.

The pharmaceutical composition may be adapted for release of the short chain fatty acids into the large intestine of the individual.

The pharmaceutical composition may be adapted for release of the short chain fatty acids into the colon of the individual.

The pharmaceutical composition may be in the form of an oral dosage form including an enteric coating which is resistant to degradation in the stomach and small intestine. The enteric coating is preferably a digestion-resistant layer on the oral dosage form designed to release the short chain fatty acids into the lumen of the large intestine, preferably the colon.

The pharmaceutical composition may in the form of an oral dosage form, a suppository, or an injectable dosage form.

The present invention also provides a dietary agent for delivery of a combination of short chain fatty acids into the large intestine of an individual, the agent including a carrier covalently bonded to the short chain fatty acids by a bond that is hydrolysable in the colon of an individual, to give free fatty acid, wherein the combination of short chain fatty acids is butyric acid and acetic acid, preferably wherein the combination further includes propionic acid, more preferably wherein the carrier is a carbohydrate selected from the group consisting of a starch, gum, oligosaccharide or pectin.

The carrier is preferably a carbohydrate selected from the group consisting of a starch, gum, oligosaccharide or pectin. More preferably, the carrier is a starch.

Where the carrier is a starch, preferably the starch is covalently bonded to at least one butyric acid and to at least one acetic acid molecule.

The invention also provides the above-mentioned dietary agent for use in the treatment of or for use in delaying the progression of an autoimmune disease selected from the group consisting of type 1 diabetes, psoriasis, rheumatoid arthritis, inflammatory bowel disease, caeliac disease, autoimmune hepatitis, myocarditis, lupus nephritis, primary biliary cirrhosis and multiple sclerosis. The dietary agent may also be for preventing or delaying the onset an autoimmune disease selected from the group consisting of type 1 diabetes, psoriasis, rheumatoid arthritis, inflammatory bowel disease, caeliac disease, autoimmune hepatitis, myocarditis, lupus nephritis, primary biliary cirrhosis and multiple sclerosis.

According to the present invention, the autoimmune disease is selected from the group consisting of type 1 diabetes, psoriasis, rheumatoid arthritis, inflammatory bowel disease, caeliac disease, autoimmune hepatitis, myocarditis, lupus nephritis, multiple sclerosis, and primary biliary cirrhosis. Preferably the autoimmune disease is type 1 diabetes.

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings.

### Brief description of the drawings

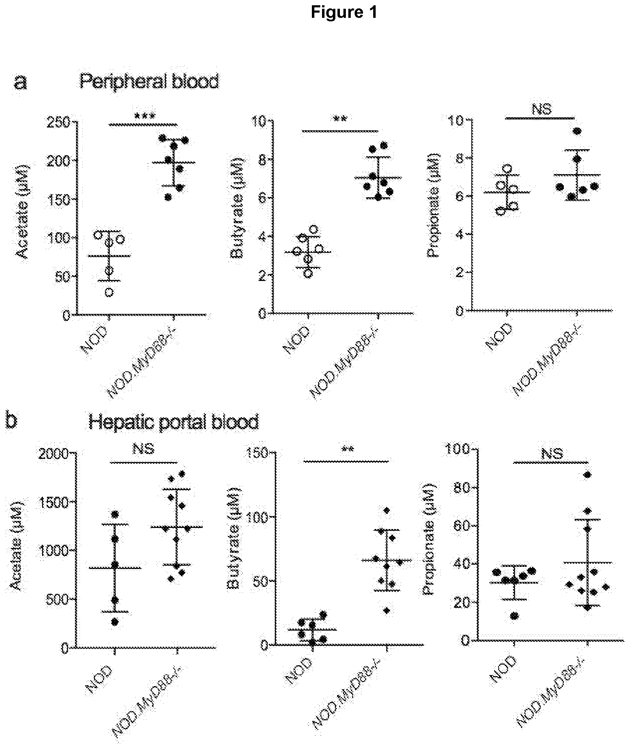

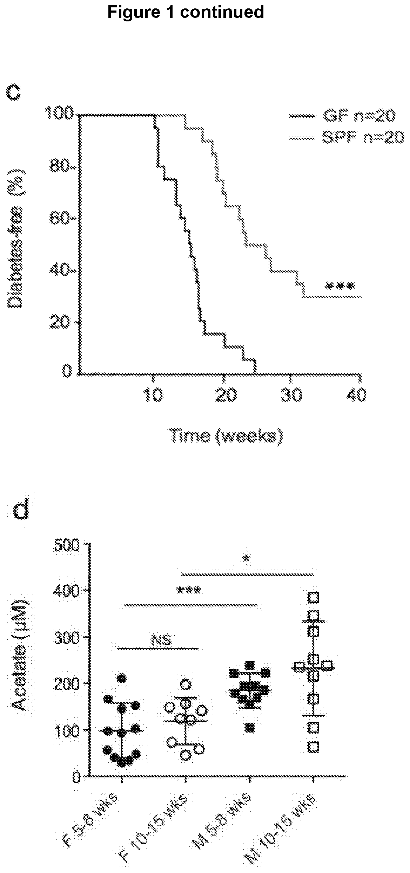

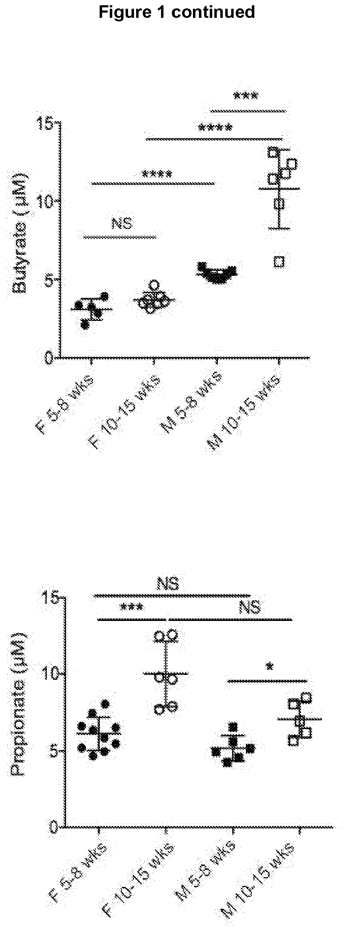

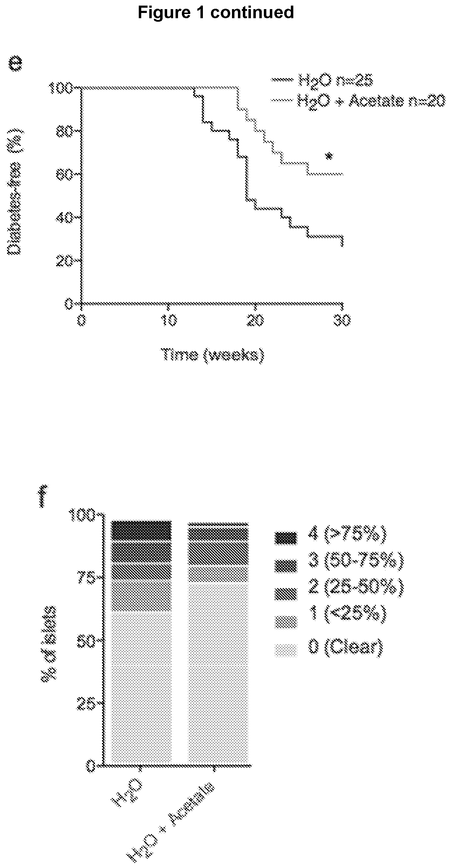

**Figure 1****.** SCFAs Concentration Correlate with Incidence of T1D in NOD Mice. Concentration of acetate, butyrate and propionate in (a) peripheral blood (vena cava) and (b) hepatic portal vein blood of 7 week-old specific pathogen-free (SPF) NOD vs. SPF NOD.MyD88-/- mice. Mann-Whitney U test. Data represent mean ± SD, n ≥ 5. Data shown is from three independent experiments. (c) T1D incidence in germ-free (GF) vs. SPF female NOD mice. ***P<0.001, Mantel-Cox log-rank test. Data shown is from two independent experiments. (d) Concentration of acetate, butyrate and propionate in peripheral blood (vena cava) of female (F) and male (M) 5-8 and 10-15 week-old NOD mice. Data represent mean ± SD, n ≥ 5. Data shown is from three independent experiments. (e) T1D incidence in SPF female NOD mice treated or untreated with 200 mM sodium acetate in drinking water (pH adjusted if needed) for 25 weeks, starting from 5 weeks of age. *P<0.0046. (f) Insulitis scores from 10 week-old NOD mice treated or untreated with acetate in drinking water. Degree of infiltration was scored as described in methods. NS= no significant. One representative experiment of two is shown.

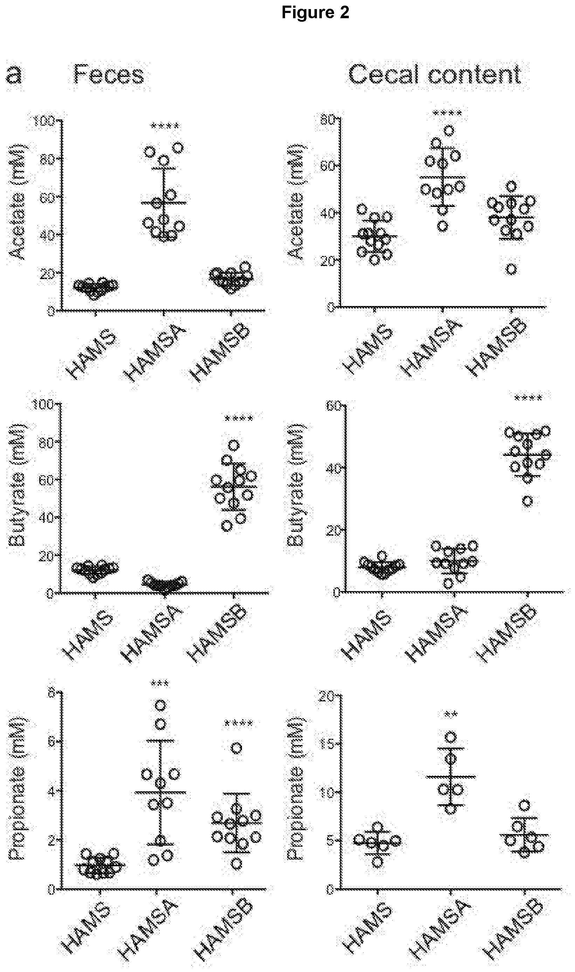

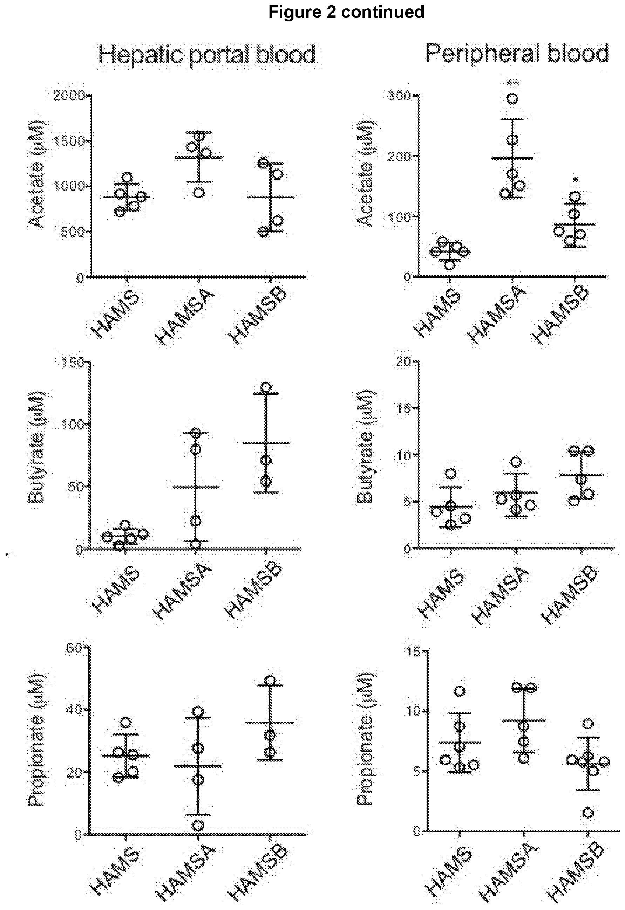

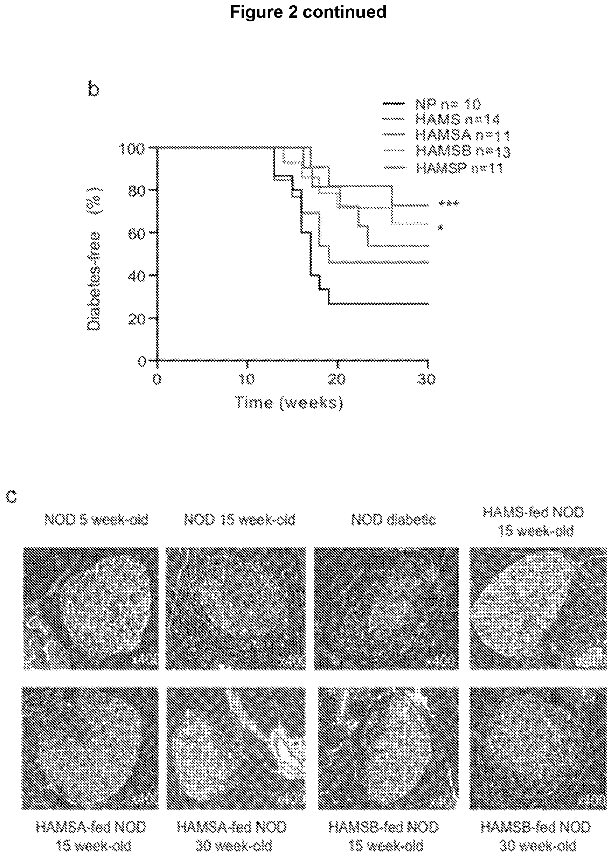

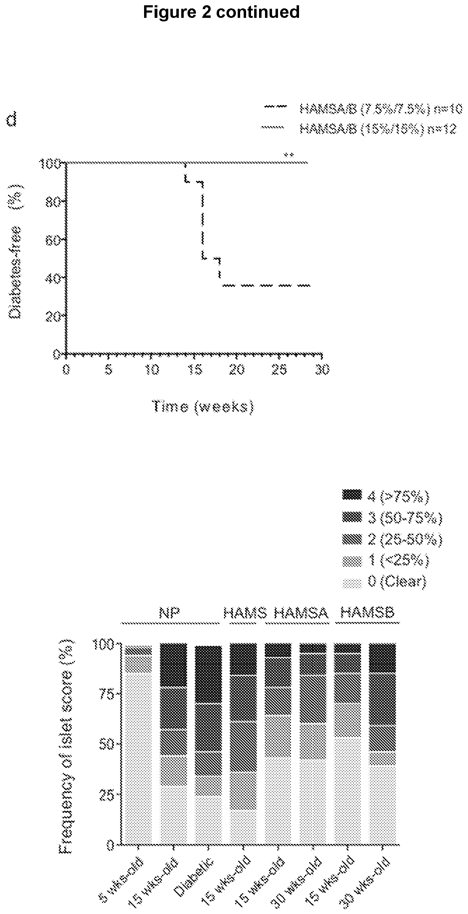

**Figure 2****.** SCFA-Delivered Diets Protect from Diabetes. (a) Concentrations of acetate, butyrate and propionate in feces, cecal contents, hepatic portal vein blood and peripheral (vena cava) blood from pooled male and female NOD mice at 15 weeks of age after 5 weeks on HAMS, HAMSA or HAMSB diet. Data shown is from three independent experiments. Data represent mean ± SD; each symbol represents biological replicates. (b) T1D incidence in female NOD mice fed NP, HAMS, HAMSA, HAMSB and HAMSP diet for a period of 5 weeks, starting at 5 weeks of age. ***P=0.0022 (HAMSA vs NP), *P=0.0476 (HAMSA vs HAMS), *P=0.042 (HAMSB vs NP) and NS (HMASP vs NP) Mantel-Cox log-rank test. (c) Representative images of islets scored for insulitis. Right hand side- Insulitis scores from female NOD mice 5 week-old, 15 week-old and 25 week-old diabetic NP-fed; 15 week-old HAMS-fed, 15 week-old HAMSA-fed and 30 week-old post-HAMSA-fed; 15 week-old HAMSB-fed and 30 week-old post-HAMSB-fed. Degree of infiltration was scored as described in methods. (d) T1D incidence in female NOD mice fed with HAMSA/HAMSB combo diet. **P=0.0018 (15%+15%, or 7.5%+7.5%) Mantel-Cox log-rank test. One representative experiment of two or three is shown. NS= no significant.

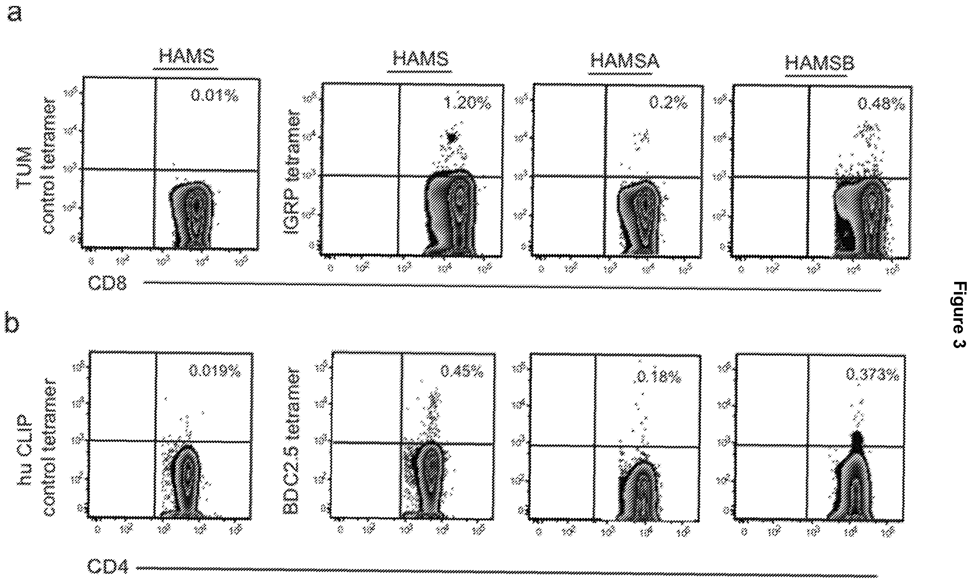

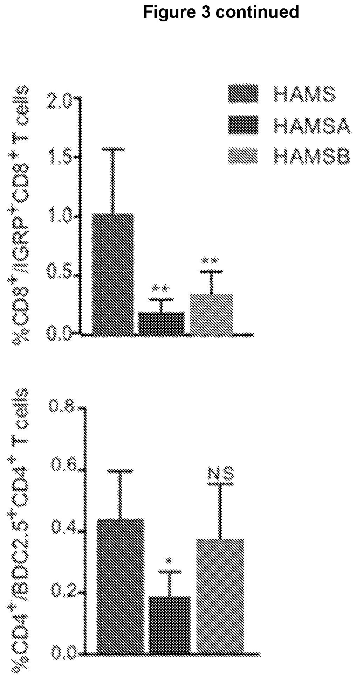

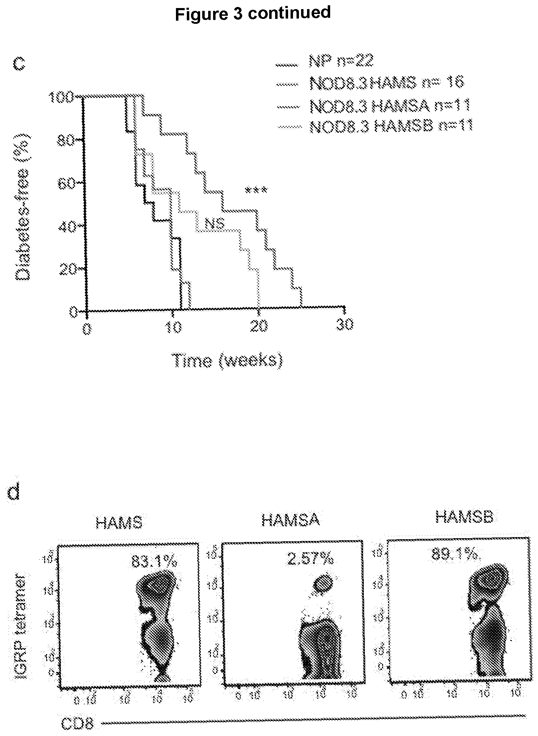

**Figure 3****.** Acetate suppresses autoimmune T cell frequencies. (a) Frequency of splenic autoreactive IGRP tetramer+ CD8+ and (b) BDC2.5 tetramer+ CD4+ T cells from 15 week-old female NOD mice fed HAMS, HAMSA or HAMSB diet. TUM and hu CLIP were used as tetramer controls respectively, n= 5-6 mice. Data shown is from three independent experiments. (c) Diabetes incidence in NOD.8.3 mice fed NP, HAMS, HAMSA or HAMSB diet. ***P<0.0001 (HAMSA vs NP). Data shown is from two independent experiments. (d) Representative plots showing frequency of IGRP tetramer+CD8+ T cells in NOD8.3 mice from (c). Data represent mean ± SD, n ≥ 5. ***P<0.001, **P<0.01, *P<0.05. Data shown is from two independent experiments.

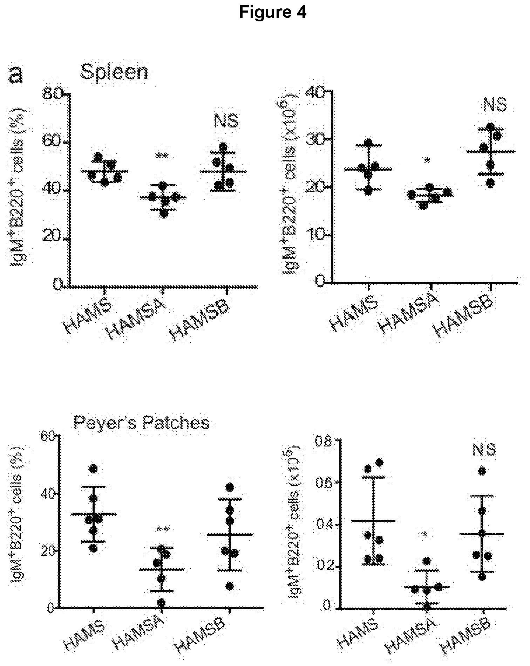

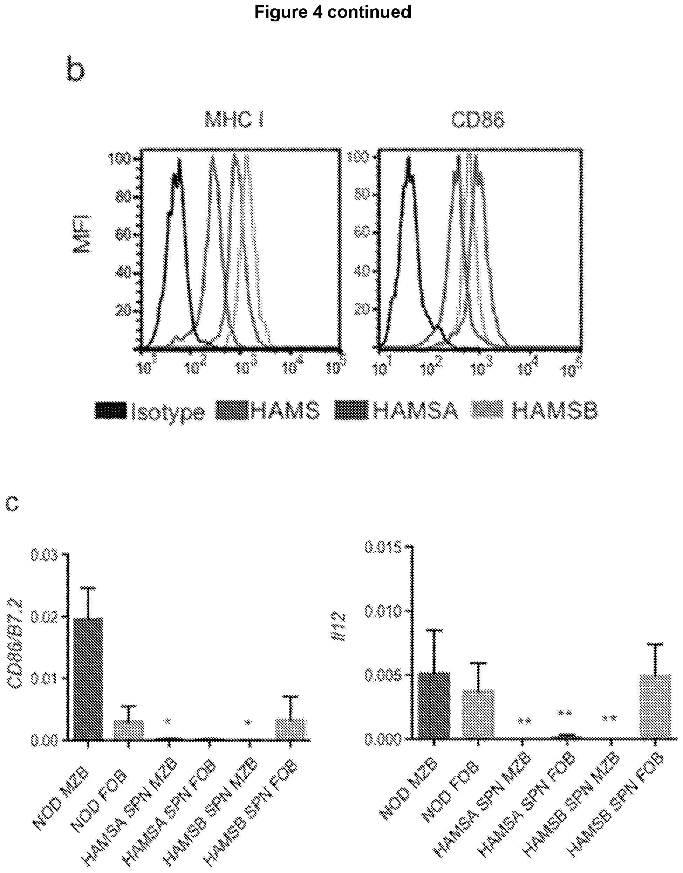

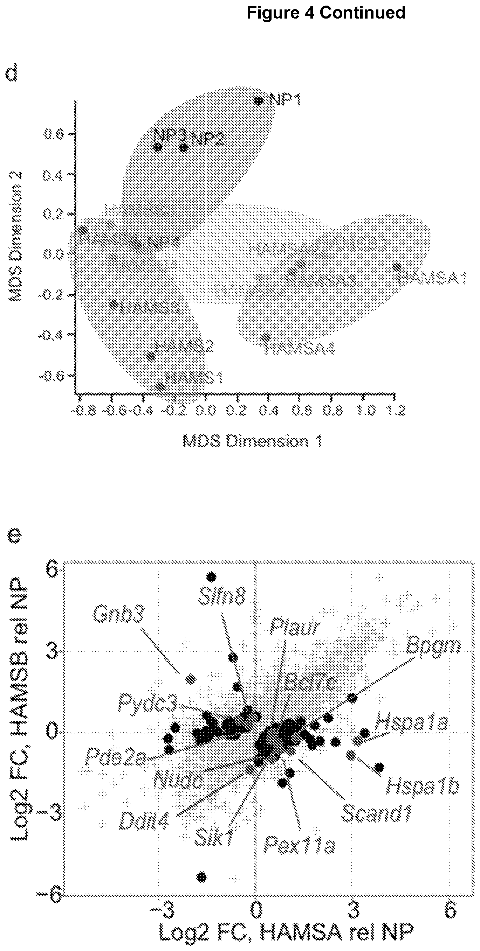

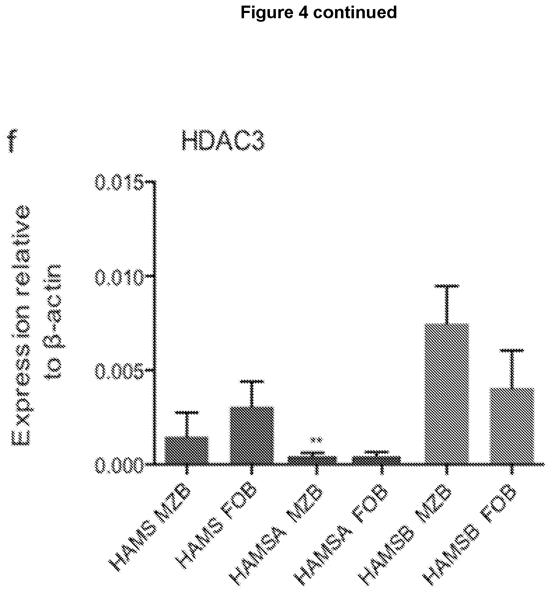

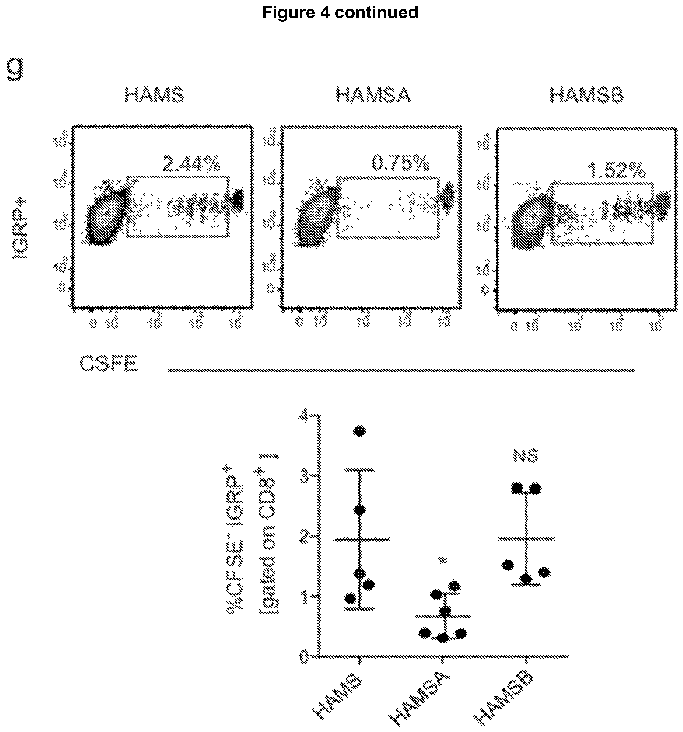

**Figure 4****.** Acetate diet affects B cell functions and gene transcription. (a) Cumulative data showing frequency and numbers of IgM+B220+ B cells in the spleen and Peyer's Patches (PP) from 15 week-old female NOD mice fed HAMS, HAMSA or HAMSB diet. (b) Representative flow cytometric analysis of MHC I and CD86 protein expression on a per-cell basis in splenic IgM+B220+ B cells from (a). Rat IgG2ak was used as isotype control (black line), n=5-6. One representative experiment of three is shown. (c) Real time PCR showing expression of CD86 and Il12 on sorted splenic CD21high CD23low (MZB) and CD21mid CD23high (FOB) cells (gated from total IgM+B220+ B cells), n=3. Data represent one of three experiments, mean ± SEM. (d) MDS plot showing differential expression from RNA-seq on IgM+B220+ B cells from (a), n=4. (e) Gene expression profile between in splenic total IgM+B220+ B cells from HAMSA vs NP contrast (x-axis), against HAMSB vs NP contrast (y-axis). Circles in red represent log2 fold-change expressed genes with FDR < 0.05 for differential expression test between HAMSA and HAMSB diets. (f) Real time PCR showing expression of HDAC3 on sorted splenic CD21high CD23low (MZB) and CD21mid CD23high (FOB) cells (gated from total IgM+B220+ B cells), P=0.0097 (HAMSA vs HAMSB) n=3. Data represent one of three experiments, mean ± SEM. (g) Proliferation of CFSE-labelled NOD.8.3 CD8+ T cells in the PLN from NP-, HAMS-, HAMSA- and HAMSB-fed NOD mice. Cumulative data showing frequency of CFSE-IGRP+CD8+ T cells in PLN. P=0.0082 (HAMSA vs HAMS). Data represent mean ± SD, n=5-6 mice. ****P<0.0001, ***P<0.001, **P<0.01, *P<0.05. One representative experiment of three is shown.

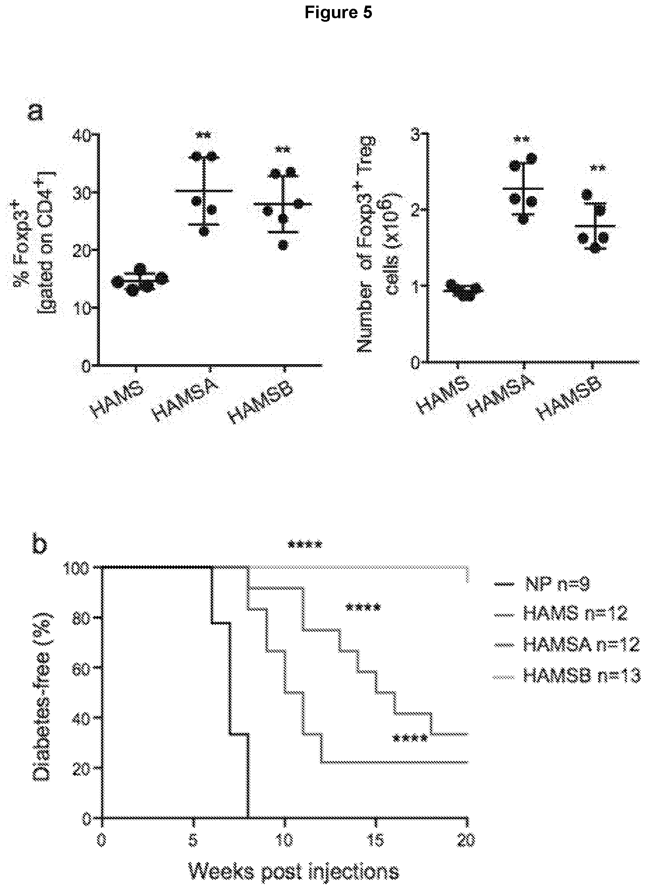

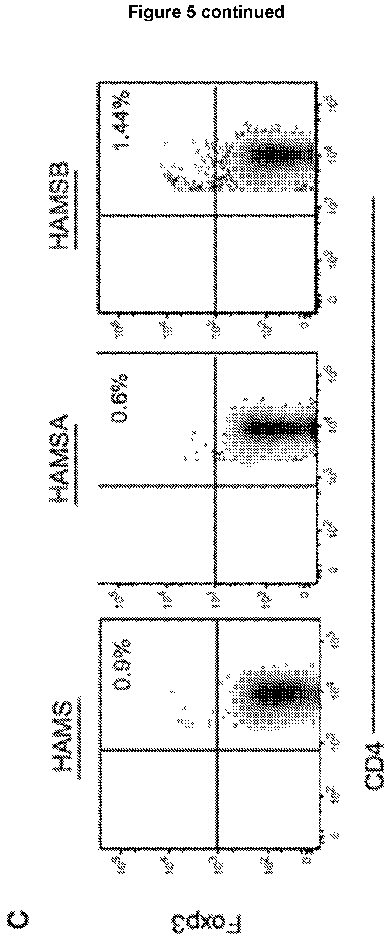

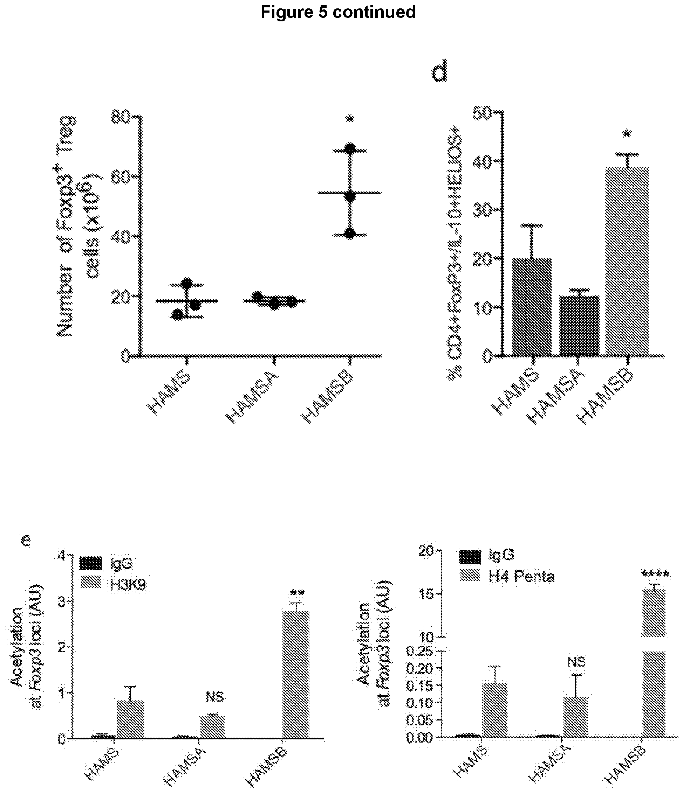

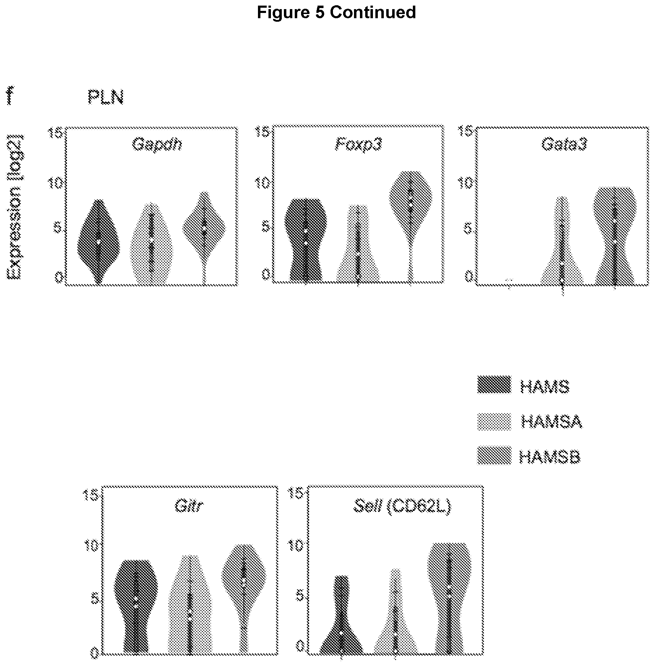

**Figure 5****.** Butyrate enhances Treg biology which contributes to protection from diabetes. (a) Cumulative data showing frequency and numbers of splenic CD4+FoxP3+ T cells from 15 week-old female NOD mice fed HAMS, HAMSA or HAMSB diet, n ≥ 5. One representative experiment of three is shown. (b) T1D incidence in NOD/SCID mice transferred with total splenic T cells from NP-, HAMS-, HAMSA- and HAMSB-fed female NOD mice. Mantel-Cox log-rank test ****P<0.0001 (HAMS vs NP-, HAMSA- vs HAMS- and HAMSB- vs HAMS-). Data shown is from two independent experiments. (c) Representative plots and cumulative data showing frequency and numbers of PLN CD4+FoxP3+ T cells 3 weeks post-transfer into NOD/SCID female fed HAMS, HAMSA or HAMSB diet. (d) Frequency of IL10-producing CD4+Foxp3+HELIOS+ T cells. Data represent mean ± SD; each symbol represents an individual mouse. ****P<0.0001, **P<0.01, *P<0.05, n=3. Data shown is from two independent experiments. (e) Acetylation of histones (H3K9 acetylation and H4 penta acetylation) at the Foxp3 promoter assessed on splenic CD4+CD25- T cells from female NOD mice fed with HAMS, HAMSA and HAMSB, n=5. P=0.0027 (H3K9, HAMS vs HAMSB) and ****P<0.0001 (H4, HAMS vs HAMSB). IgG isotype control was used to assess unspecific binding. Arbitrary unit (AU) represents enrichment of acetylation relative to H3 binding. (f) Single-cell expression of Foxp3, Gata3, Gitr and Sell (CD62L) in CD4+ T cell expressing CD45RBlowCD25+ from PLN of female NOD mice fed HAMS, HAMSA or HAMSB diet. Single cells obtained from pooled mice, n=6. Data shown is from three independent experiments. (g) Venn diagrams of CD4+CD45RBlowCD25+Foxp3high PLN T cells (from B) showing co-expression of Gata3, Gitr and Sell (CD62L) in HAMS-(red venn diagrams) and HAMSB-fed mice (green venn diagrams). The numbers inside the venn diagrams represent the number of Foxp3high cells (filled circles) that express Gata3, Gitr and Sell (CD62L), n=30 cells individually sorted.

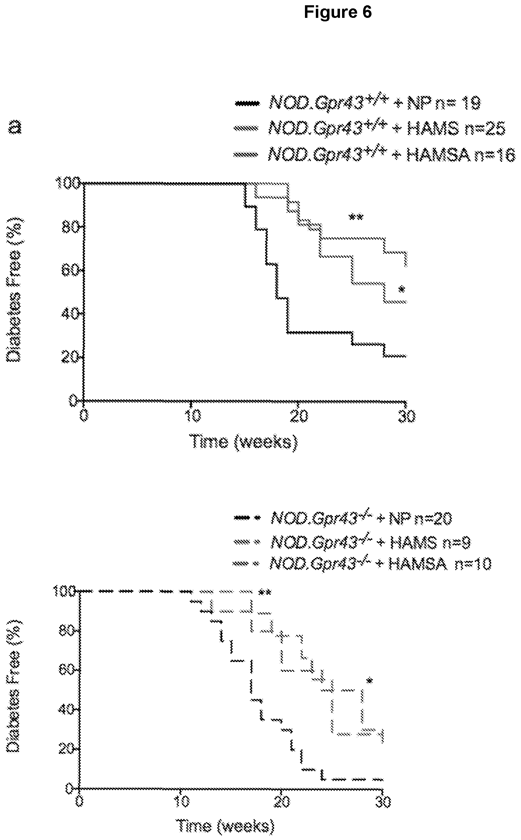

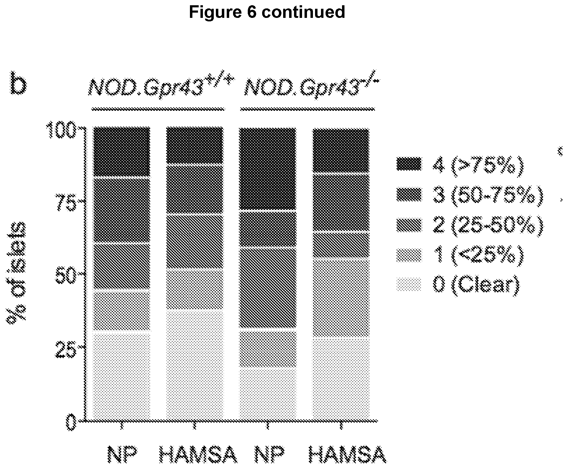

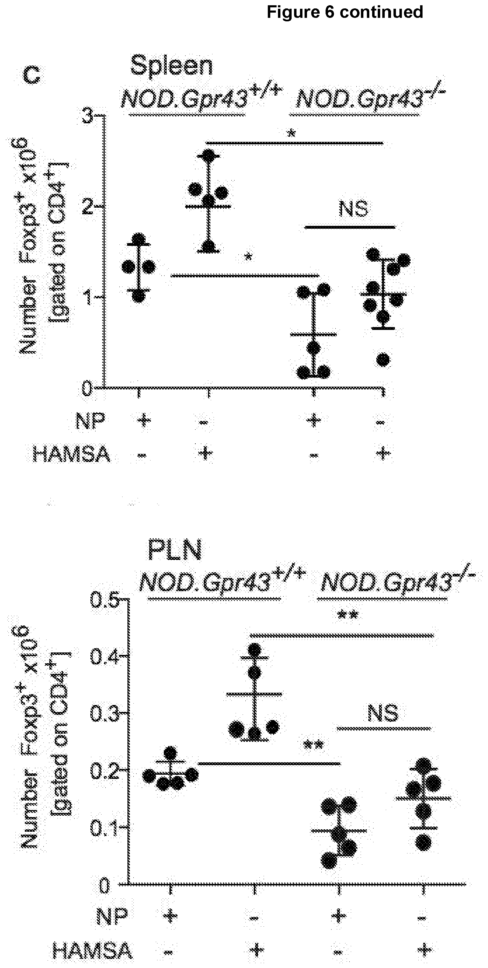

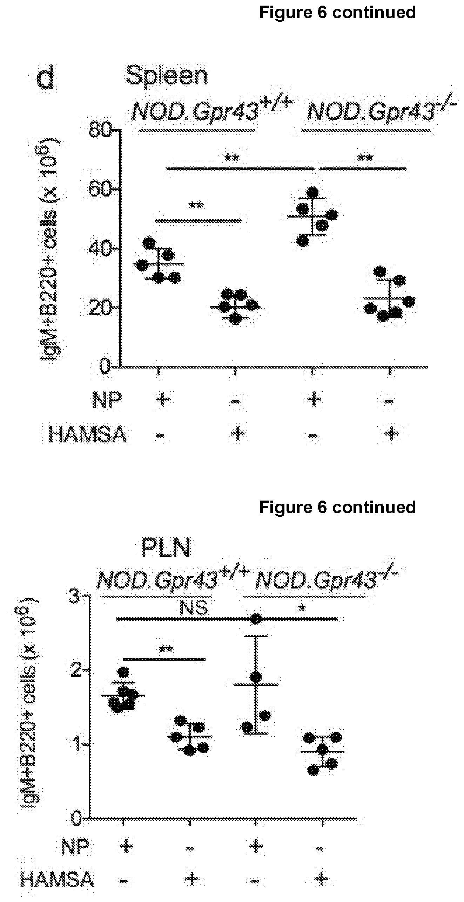

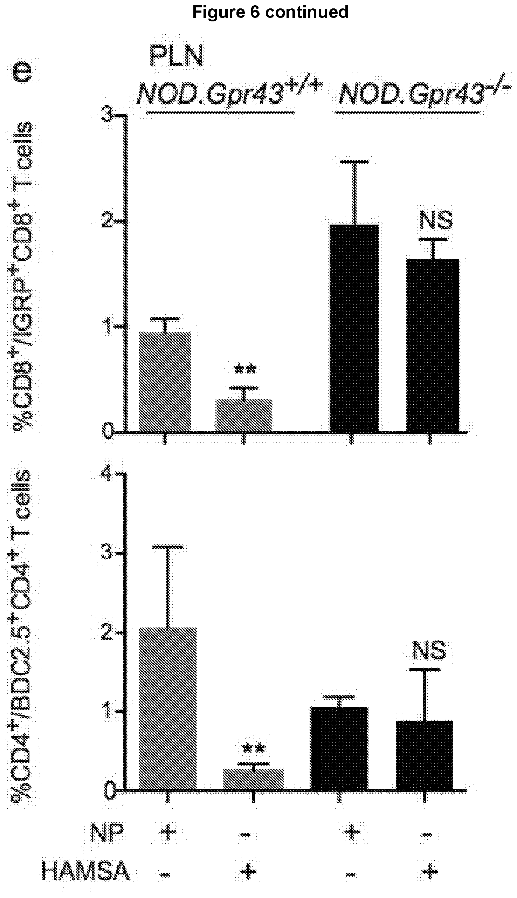

**Figure 6****.** Acetate acts in part through GPR43 to limit T1D Severity. (a) T1D incidence in female NOD.Gpr43+/+ and NOD.Gpr43-/- littermates fed NP, HAMS or HAMSA diet. **P=0.0023 (NOD.Gpr43+/+ mice, NP vs HAMSA); *P=0.0392 (NOD.Gpr43+/+ mice, NP_vs HAMS) and *P=0.0179 (NOD.Gpr43-/- mice, NP vs HAMSA). Data shown is from two independent experiments. (b) Insulitis scores from 15 week-old NOD.Gpr43+/+ and NOD.Gpr43-/- mice fed NP or HAMSA diets. Degree of infiltration was scored as described in methods. (c) Cumulative data showing absolute numbers of splenic and PLN CD4+FoxP3+ T cells and (d) IgM+B220+ B cells, n ≥ 5. Data shown is from three independent experiments. (e) Frequency of PLN autoreactive IGRP tetramer+ CD8+ and IAg7/BDC2.5 tetramer+ CD4+ T cells from 15 week-old female NOD.Gpr43+/+ and NOD.Gpr43-/- mice fed NP or HAMSA diet. Data represent mean ± SD, n ≥ 4. Data shown is from three independent experiments.

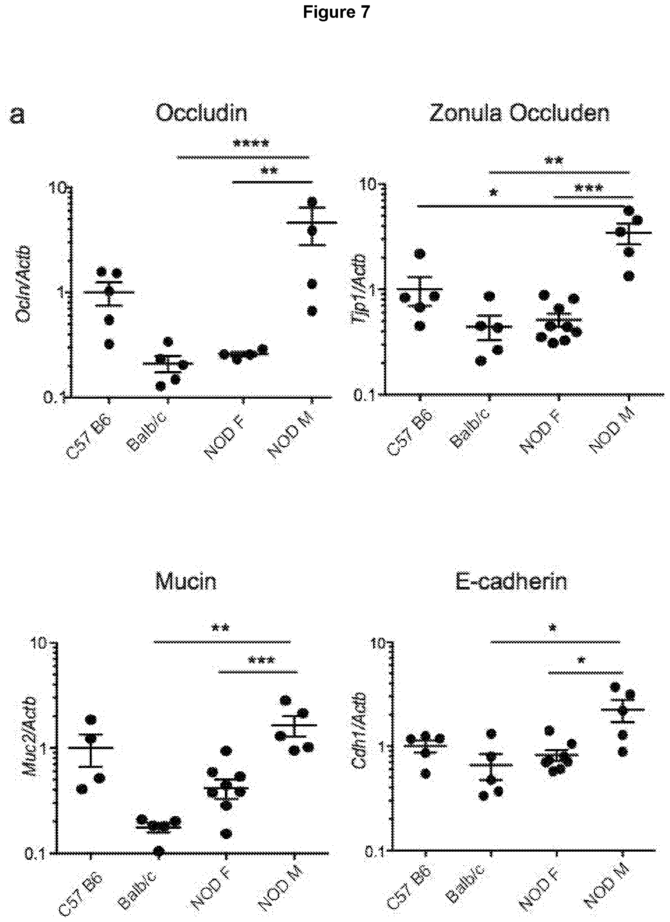

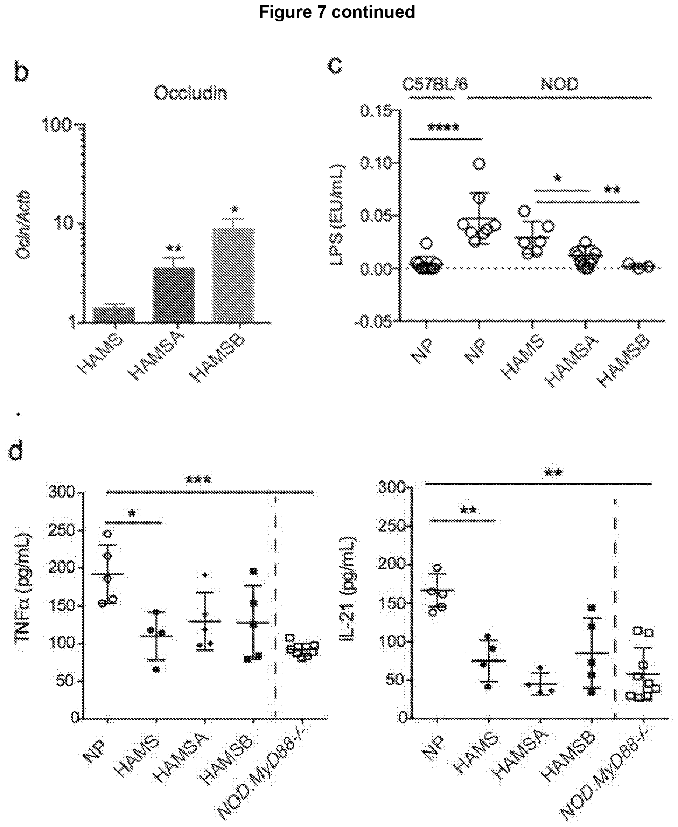

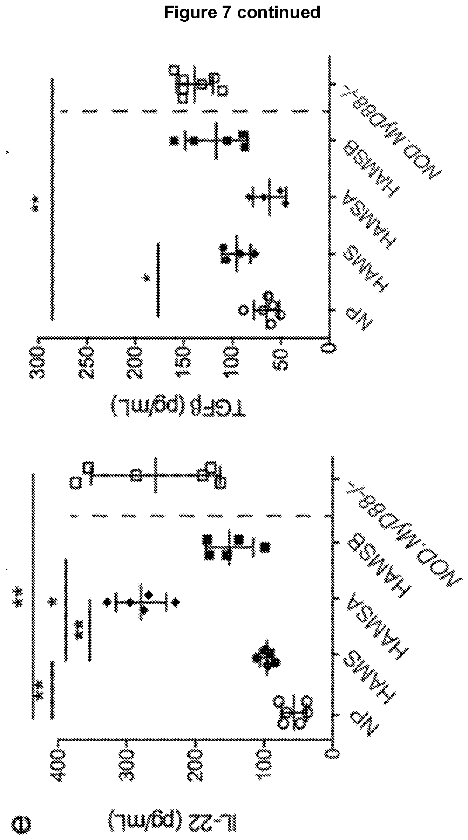

**Figure 7****.** Acetate and butyrate diets improve parameters important in T1D pathogenesis, including LPS, IL-21 and TNFα. **(a)** Fold change expression of *Ocln*, *Tjp1, Muc2* and *Cdh1*, mRNA in the colon of 5 week-old NP-fed female C57BL/6, Balb/c mice and age-matched NP-fed female (F) and male (M) NOD mice. No gender differences were shown in control C57BL/6 and Balb/c mice, n ≥4 biological replicates in each diet. **(b)** Fold change in expression of *Ocln* in the colon of 15 week-old HAMS-, HAMSA-, or HAMSB-fed female NOD mice. Data present mean ± SEM, n ≥ 7 biological replicates in each diet. Data shown is from three independent experiments. (c) Concentration of LPS in peripheral blood (vena cava) of female age-matched NP-fed C57BL/6 and female NOD mice fed NP, HAMS, HAMSA or HAMSB diet. Data present mean ± SD, n ≥3 biological replicates in each diet. Data shown is from three independent experiments. Concentrations of **(d)** TNF-α, IL-21, **(e)** IL-22 and TGF-β in serum from 15 week-old HAMS-, HAMSA-, or HAMSB-fed female NOD mice compared to age-matched NP-fed *NOD.MyD88*^{*-*/*-*} mice, n ≥4 biological replicates in each diet. Data represent mean ± SD; each symbol represents an individual mouse. ****P<0.0001, **P<0.01, *P<0.05. Data are from three independent experiments.

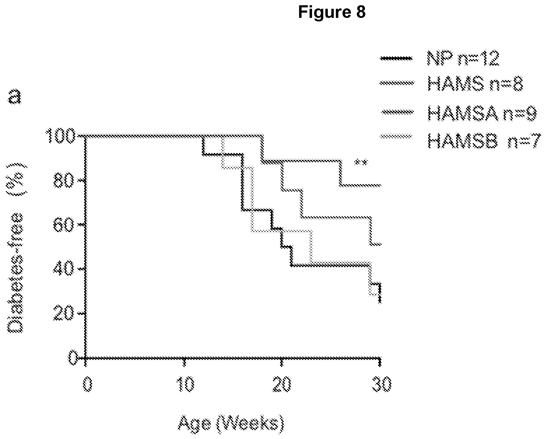

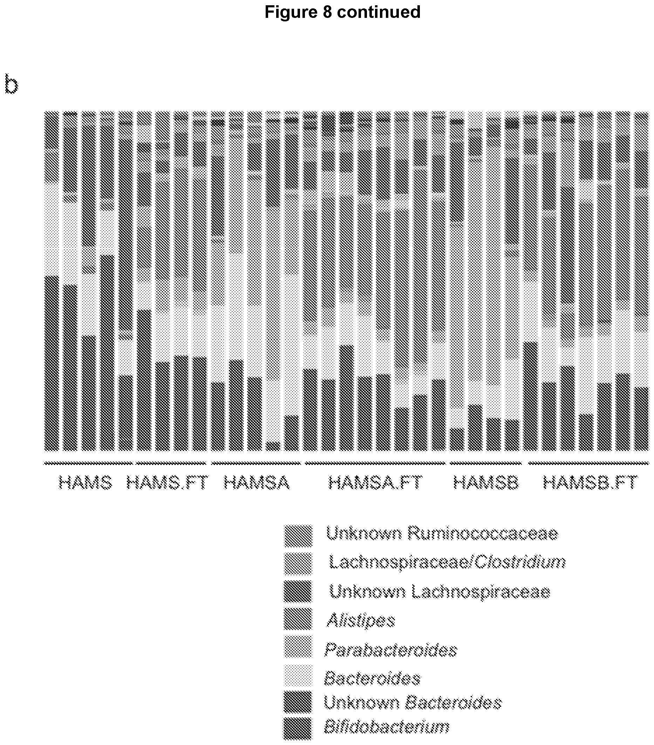

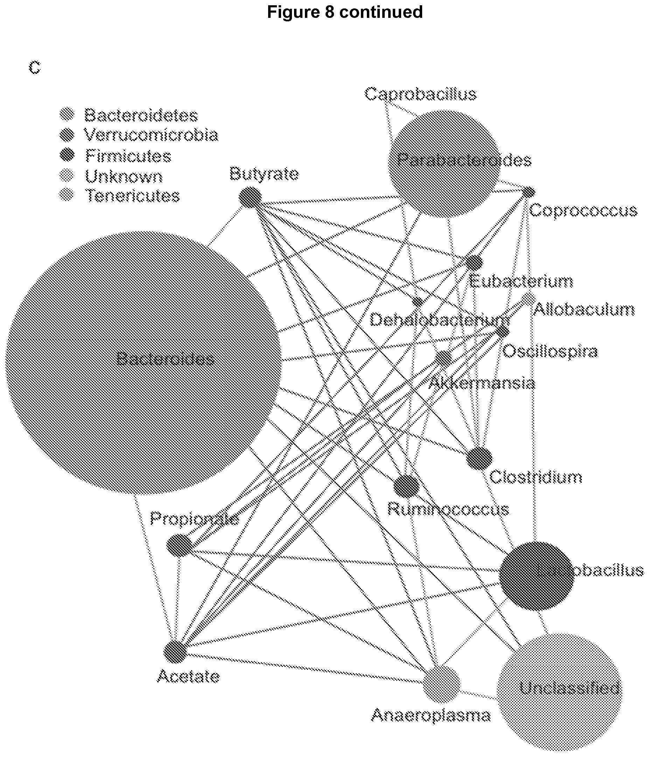

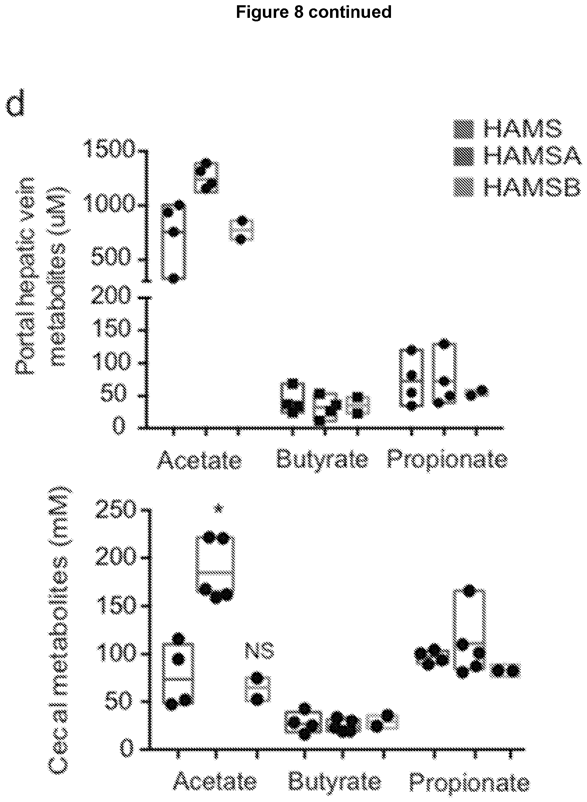

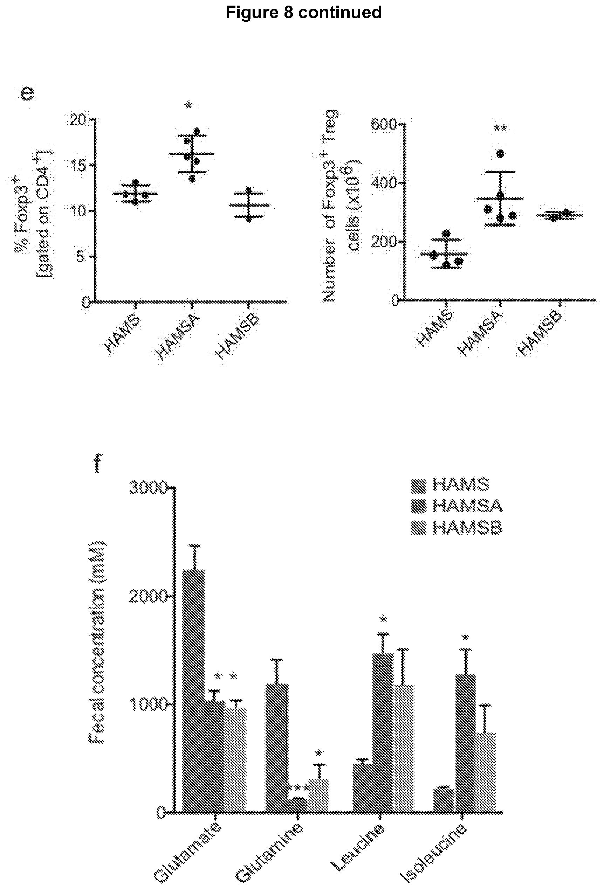

**Figure 8****.** Diets alter microbial ecology and metabolite production, which contributes to diabetes protection. (a) T1D incidence in female GF NOD mice re-colonized with NP-, HAMS-, HAMSA- or HAMSB-shaped microbiota. *P=0.0204 (HAMSA vs NP) Mantel-Cox log-rank test. (b) Bar chart showing distribution of genera detected in feces from SPF NOD mice and GF NOD mice after fecal transfer (FT) for different diets, n=5-6 per group. Each genus is represented by a different colour and is proportional to the relative abundance in each sample. The legend shows the genera with relative abundance higher than 1%. (c) Pearson correlation-based network showing relationships between SCFAs acetate, butyrate and propionate and bacterial genera in NOD mice fed NP, HAMS, HAMSA or HAMSB diet. Genera-depicting nodes are colored by phylum that genus belongs to while acetate, butyrate and propionate are represented with red circles. Size of each genus node is proportional to relative abundance of that genus; green lines connect positively and blue lines negatively correlated nodes. Genus *Bacteroides*, positively correlated with both acetate and butyrate, thrived in high acetate and butyrate environments and likely suppressed growth of the module of other, positively correlated, (green lines) genera. (d) Cumulative data showing serum and cecal concentrations of acetate, butyrate and propionate from 30 week-old GF re-colonized NOD mice from (a), n ≥ 2. Data presented as mean from two independent experiments. (e) Frequency and number of PLN CD4+Foxp3+ Treg cells from 30 week-old GF NOD mice re-colonized with diet modified-microbiota. (f) Concentration of fecal metabolites in 15 week-old female NOD mice fed HAMS, HAMSA or HAMSB diet, n ≥ 5. (f) Concentration of fecal metabolites in protected 15 week-old female NOD mice fed HAMS, HAMSA or HAMSB diet, n ≥ 2-5. Data presented as mean ± SEM. Symbols represent individual mice. ***P<0.001, **P<0.01, *P<0.05. Data represent the combination of two independent experiments.

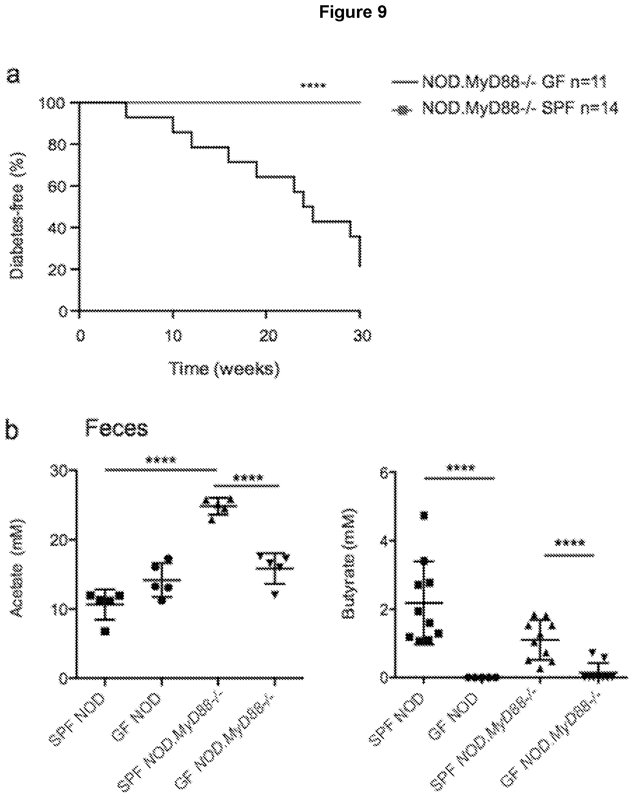

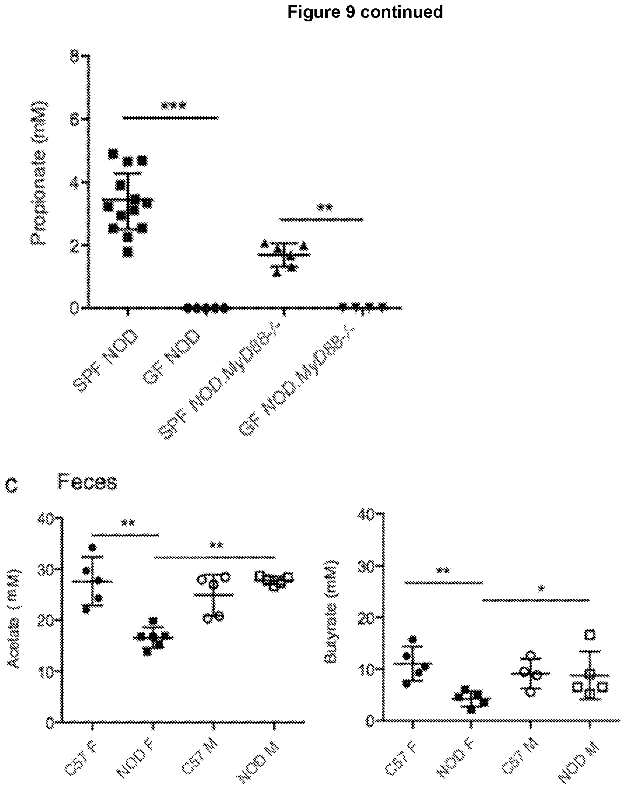

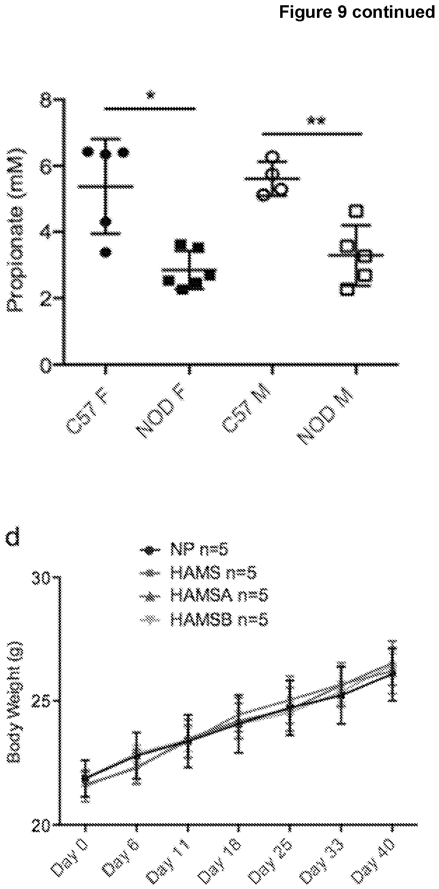

**Figure 9****.** (a) T1D incidence in germ-free (GF) NOD.MyD88-/- mice vs. specific pathogen-free (SPF) NOD.MyD88-/- mice; ****P<0.0001, Mantel-Cox log rank test. Concentrations of acetate, butyrate and propionate in (b) feces of 5 week-old female SPF and GF NOD and NOD.MyD88-/- mice and (c) Concentrations of acetate, butyrate and propionate in feces of age matched female and male NOD and C57BL/6 mice. For all graphs, data represent mean ± SD; each symbol represents an individual mouse. (d) Body weights of 15 week-old female NOD mice fed with NP, HAMS, HAMSA or HAMSB diets. ****P<0.0001, **P<0.01, *P<0.05. All data are representatives of three independent experiments.

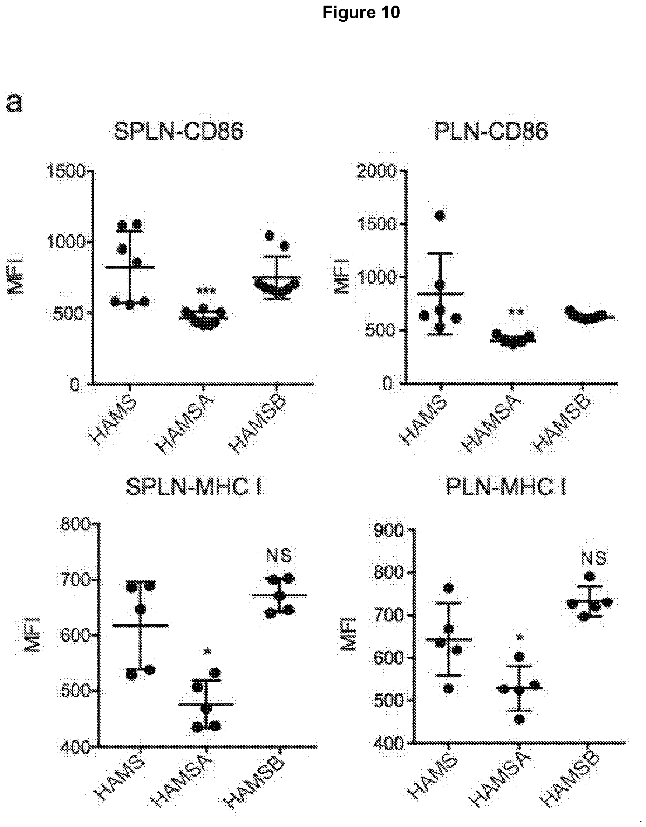

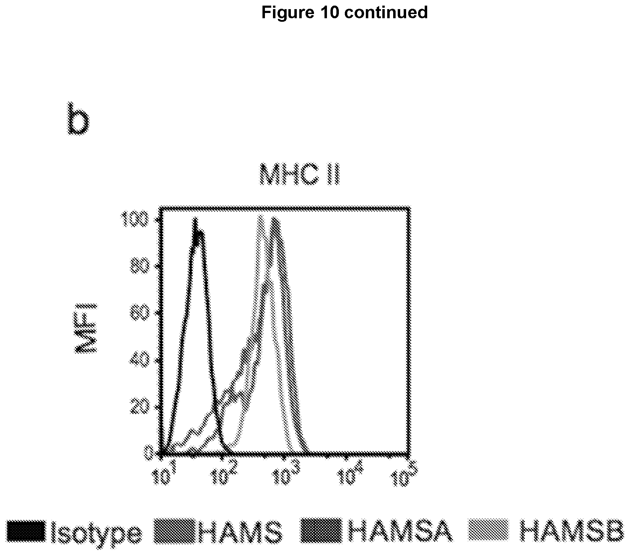

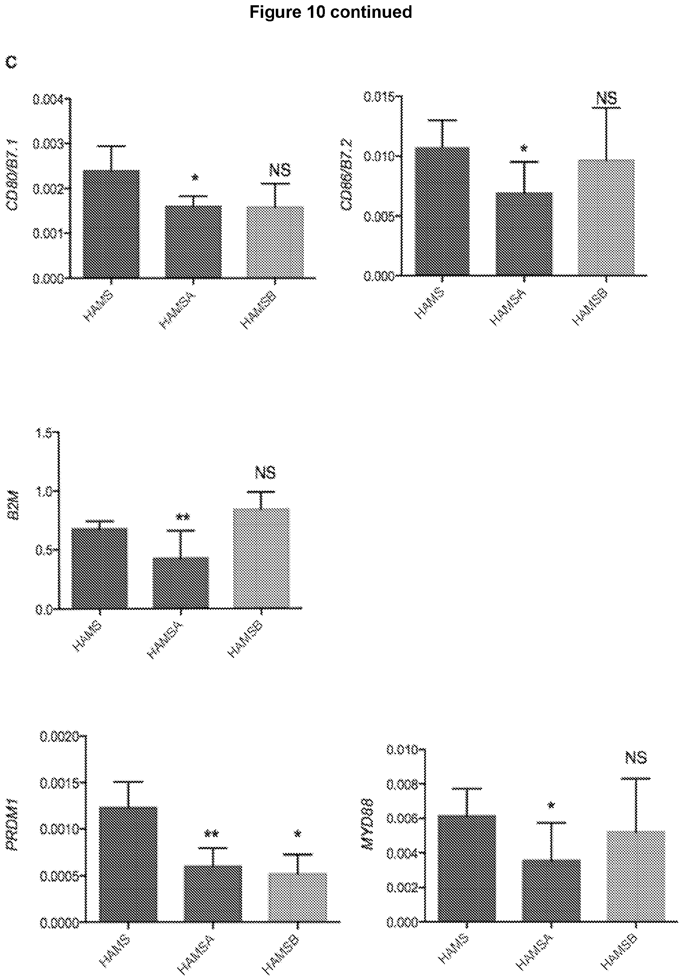

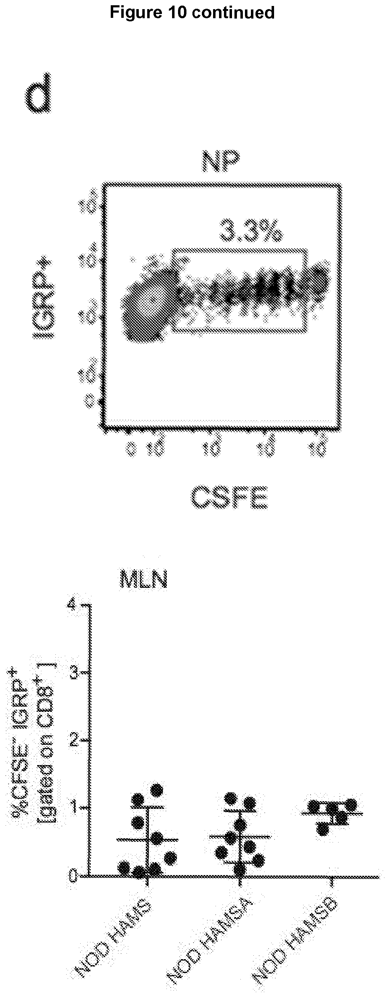

**Figure 10****.** (a, b) Cumulative data as mean fluorescence index (MFI) +/- DS of surface protein expression for MHCI, CD86 and MHCII on a per-cell basis in splenic IgM+B220+ B cells from (Fig. 4a). (c) Real time PCR showing fold change in expression of C80/B7.1, C80/B7.2, B2M and PRDM1 relative to β-actin in splenic IgM+B220+ B cells from of 15 week-old HAMS-, HAMSA-, or HAMSB-fed female NOD mice. (d) Representative FACS plot and cumulative data showing frequency of transferred CFSE-IGRP+CD8+ T cells from total CD8+ T cells isolated from the MLN of NP-fed NOD mice, HAMS-, HAMSA- or HAMSB-fed NOD mice. Data present mean ± SD, n ≥ 6 biological replicates in each diet. Data are representative of at least three independent experiments.

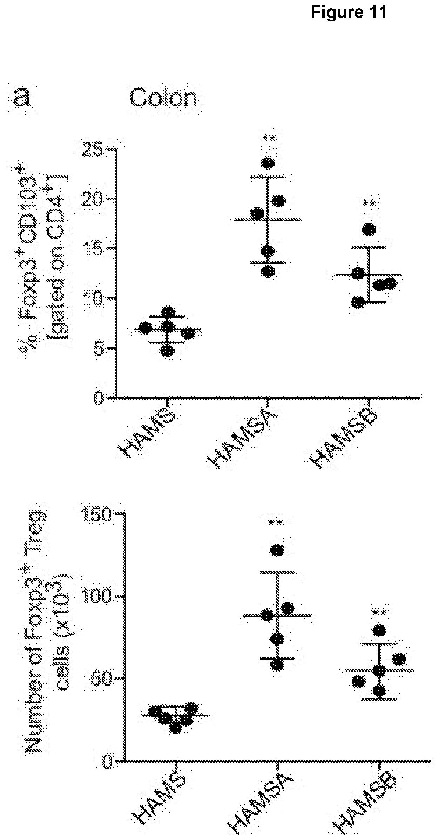

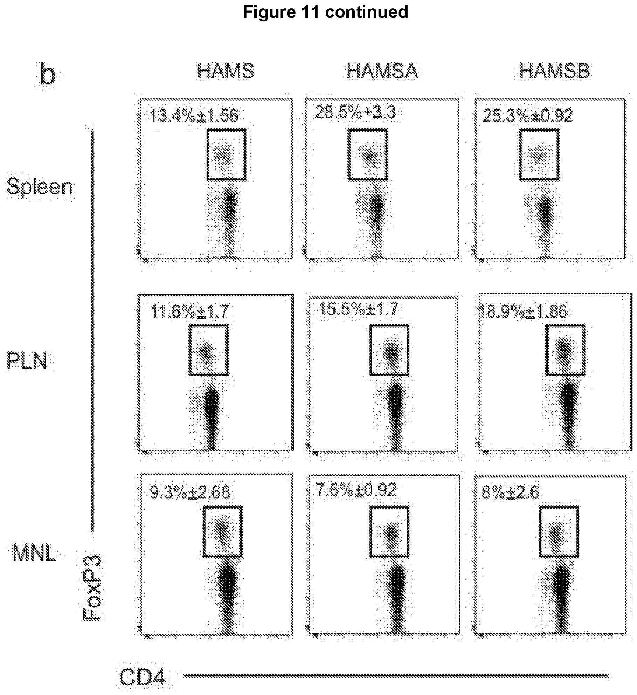

**Figure 11**. (a) Cumulative data showing frequency and numbers of CD4+FoxP3+CD103+ T cells from total CD4+ T cells isolated from the colon of 15 week-old female NOD mice fed HAMS, HAMSA or HAMSB diet. (b) FACS plots showing frequency of splenic, PLN and MNL CD4+FoxP3+ T cells. Data represent mean ± SD; each symbol represents an individual mouse. **P<0.01. All data are representatives of three independent experiments.

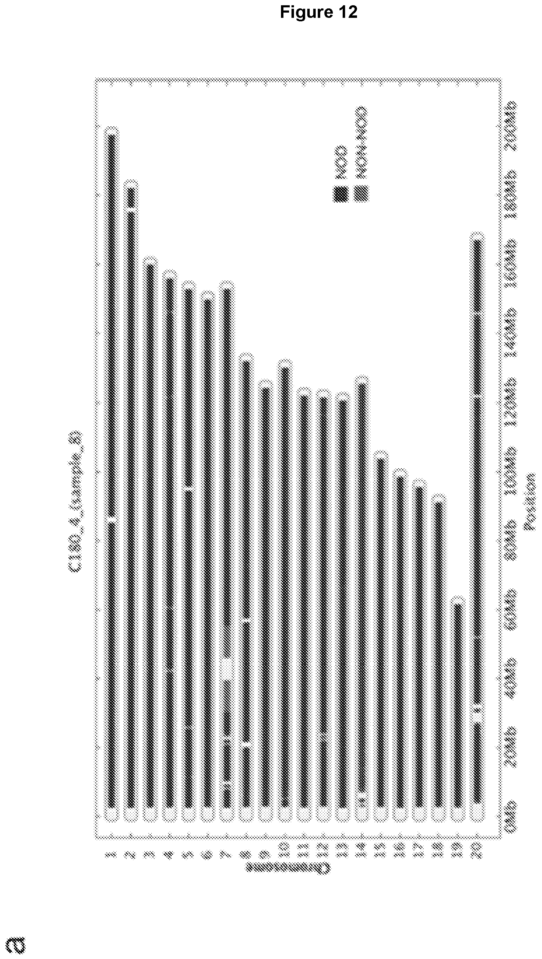

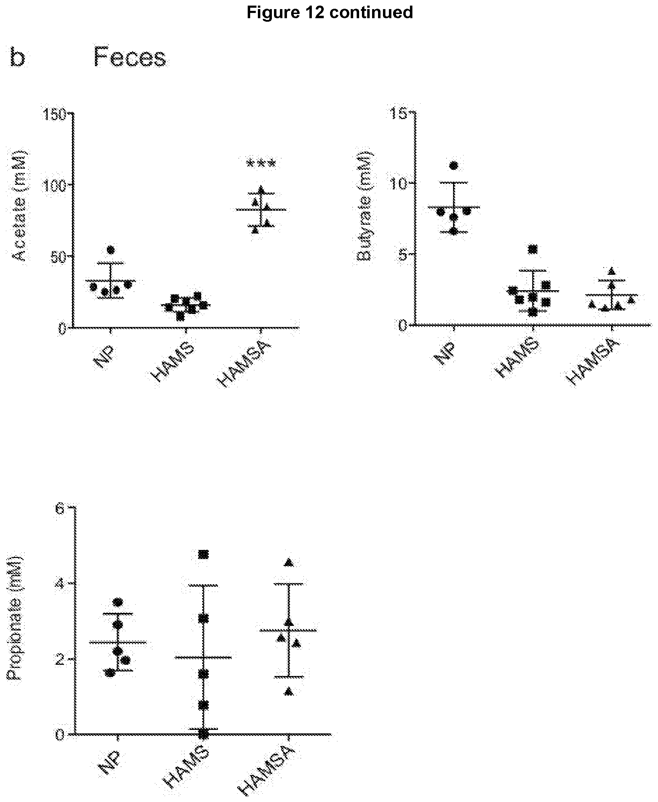

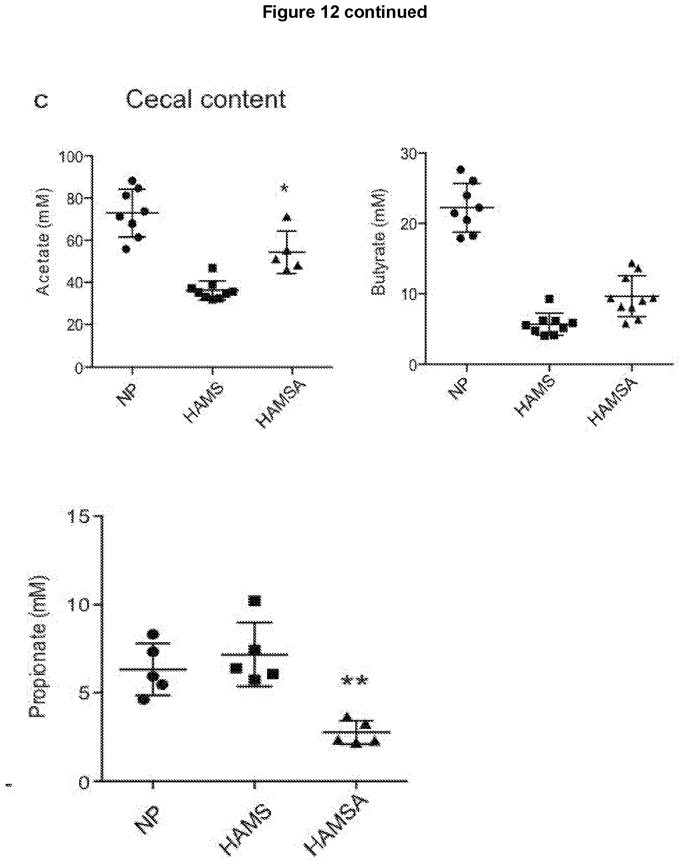

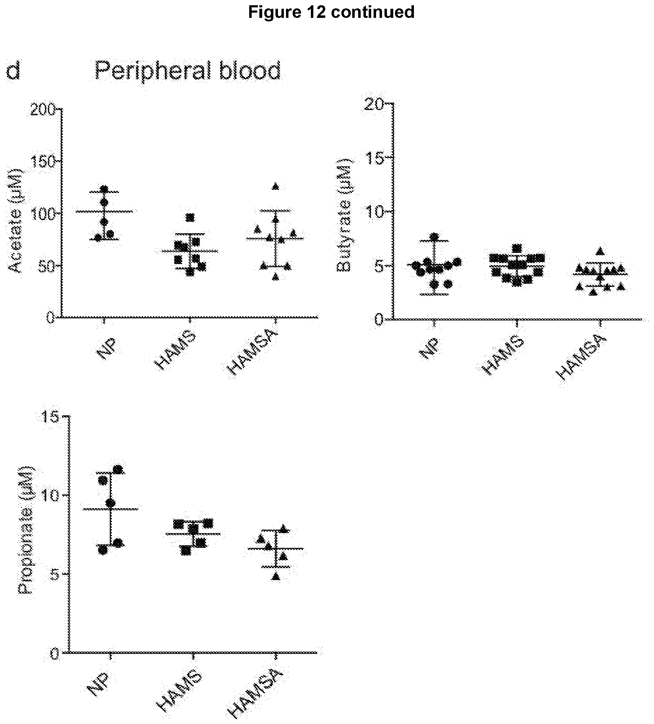

**Figure 12**. (a) C57.Gpr43-/- mice were backcrossed 13 generations onto the NOD strain (NOD.Gpr43-/-). Once fully backcrossed, NOD.Gpr43-/- mice were genotyped at over 70,000 SNPs genome-wide. DNA from liver was purified and genotyped using the Mega-MUGA array (Geneseek, NB). Genotypes were compared to the reference (C57BL/6) alleles and to NOD alleles determined by the NOD genome sequence (Yalcin et al., 2011). The knockout was produced on the FVB background, so haplotypes are depicted as coming from the NOD genome (blue) or non-NOD genomes (red); grey indicates non-informative regions in which C57BL/6 and NOD have the same genotypes. This figure shows the strain of origin of haplotypes on each mouse chromosome, with the physical size of each chromosome shown on the X axis. This analysis demonstrated that all chromosomes were derived from the NOD strain, except for a region around the Gpr43 locus on chromosome 7. Thus, these mice harbour all NOD T1D susceptibility loci including the Idd7 and Idd27 loci mapped to ~19Mb and ~80-120 MB on chromosome 7, respectively. This enables us to confirm that there have been no T1D susceptibility genes reported in the non-NOD interval. (For all graphs, each symbol represents an individual mouse. (Concentrations of acetate, butyrate and propionate in (b) feces, (c) cecal content and (d) peripheral blood (vena cava) of 15 week-old female NOD.Gpr43-1- mice fed NP, HAMS or HAMSA diet. ***P<0.001, **P<0.01, *P<0.05 (HAMSA- VS HAMS-fed NOD.Gpr43-/- mice). Each symbol represents an individual mouse. Data presented as the mean ± SD. All data are representatives of three independent experiments.

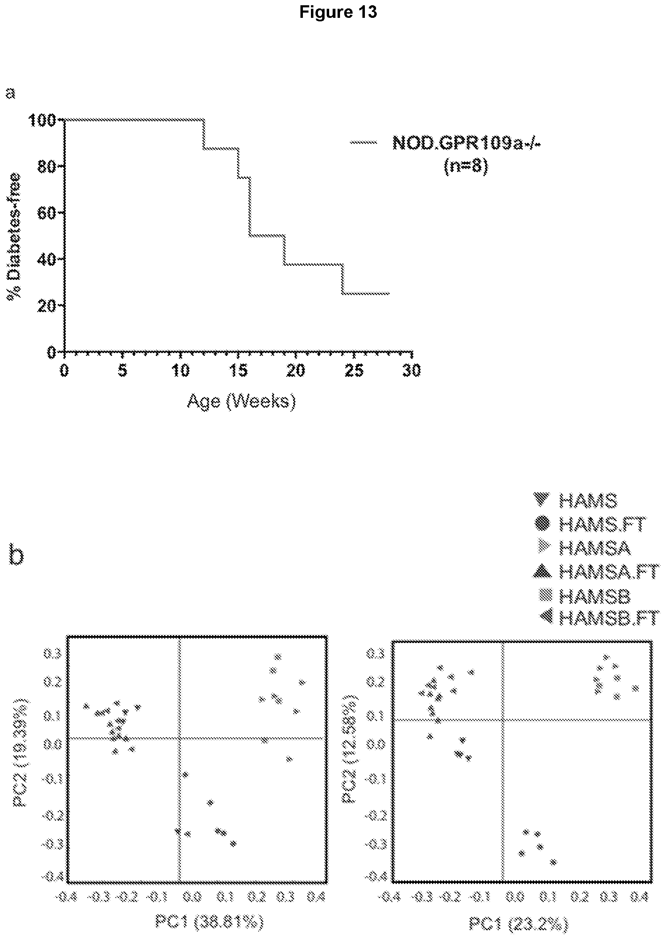

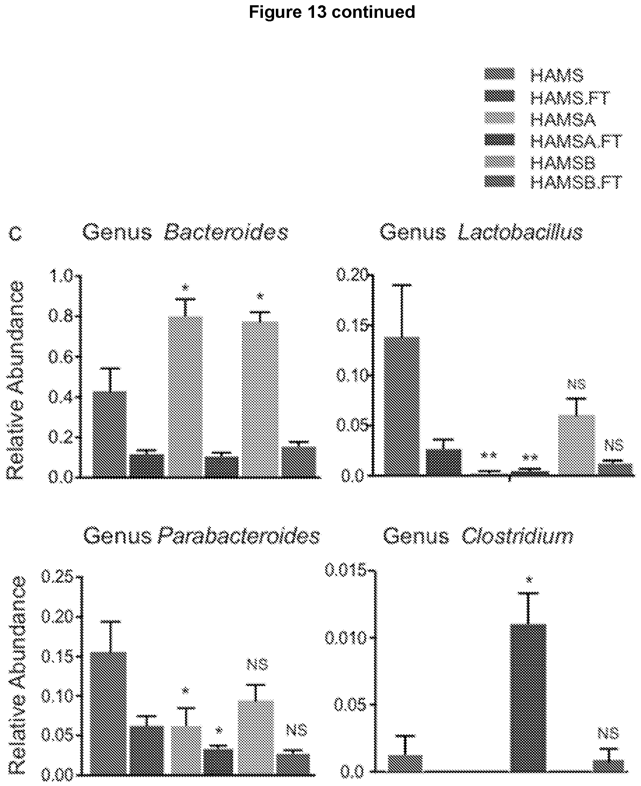

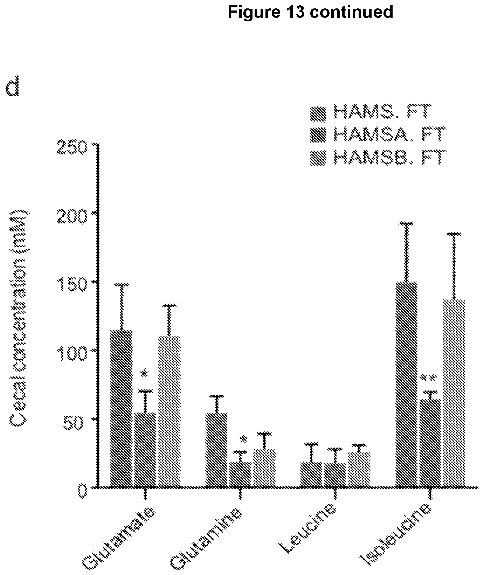

**Figure 13****.** (a) T1D incidence in female NOD.Gpr109a-/- fed NP. (b) Microbial profile analysis of feces from NOD and GF re-colonized NOD mice fed NP, HAMS, HAMSA and HAMSB diet by PCoA diagrams based on unweighted (left panel) and weighted (right panel) Unifrac distance metrics. The bacterial communities of different diets were significantly different based on both weighted (P= 6.2E-5) and unweighted (P<1E-6) Unifrac, 1E6 permutations and Adonis permutational multivariate statistics. The legend represents the microbiota from donors NOD mice and GF NOD mice re-colonized with NP, HAMS, HAMSA and HAMSB modified microbiota in the same colour, with GF re-colonized NOD mice in darker shade. (FT) indicating fecal transplant. (c) Relative abundance of selected bacterial populations at (genus level) in NOD mice fed NP, HAMS, HAMSA or HAMSB diet and in GF NOD mice after fecal transfer (FT) for different diets. Data represent mean ± SD; each symbol represents individual mice. *P<0.05. All data are representatives of two independent experiments.

### Detailed description of the embodiments

Increased fiber consumption as part of a healthy diet is recognised for its potential to protect against chronic disease, particularly consumption of resistant starches that are fermented by the colonic microbiota (Macia et al., 2015). This fermentation produces short chain fatty acids (SCFAs) principally acetate, propionate and butyrate. It is likely that SCFAs mediate many of the effects ascribed to fiber, and their supply is critical for optimal gut function.

SCFAs produced from bacterial fermentation of fiber in the large intestine may promote gut health in numerous ways. For example, these acids are thought to be important for maintaining visceral function by increasing blood flow, and contribute to improved electrolyte and fluid absorption in diarrhea, maintenance of low colonic pH to limit the growth of intestinal pathogens and also the modulation of colonic muscular activity. However, while diets high in acetylated or butyrylated high amylose maize starches (known as HAMSA and HAMSB, respectively) have been trialled in human populations with GI tract disorders (Annison et al., 2003, Feehily & Karatzas, 2013), most efforts described previously for the correction of dysbiosis have relied on the use of probiotics.

The present inventors have surprisingly found that SCFAs also play a role outside of the digestive system and in particular, an important role in the protection against autoimmune disease. Specifically, the inventors have found that providing a combination of at least two different SCFAs protects against the development of autoimmune disease and may also represent a novel treatment modality.

Accordingly, in a first aspect, the present invention relates to a therapeutically effective amount of a combination of two or more short chain fatty acids, esters or salts thereof, for use in treating an autoimmune disease or for use in delaying or preventing the progression of an autoimmune disease in an individual, wherein the combination of short chain fatty acids is butyric acid and acetic acid, preferably wherein the combination further includes propionic acid, and wherein the short chain fatty acids are conjugated to a carrier in the form of a starch molecule.

The invention also provides a therapeutically effective amount of a combination of two or more short chain fatty acids, esters or salts thereof, for use in preventing or delaying the onset of autoimmune disease in an individual, wherein the combination of short chain fatty acids, esters or salts thereof, is butyric acid and acetic acid or esters or salts thereof, preferably wherein the combination further includes propionic acid, and wherein the short chain fatty acids are conjugated to a carrier in the form of a starch molecule

In a particularly preferred embodiment, the SCFAs are butyric acid and acetic acid. In yet a further embodiment, all three species of SCFA are utilised.

The short chain fatty acids may be provided as sodium, potassium, calcium or magnesium salts. Where one of the two or more short chain fatty acids is butyric acid, preferably, the salt is sodium butyrate. Where one of the two or more short chain fatty acids is acetic acid, preferably the salt is sodium acetate.

For example, the short chain fatty acid can be present as an ethyl ester, propyl ester, butyl ester, isopropyl ester or t-butyl ester.

When one of the two or more short chain fatty acids is butyric acid, butyric acid is provided as a prodrug in the form of tributyrin, which is an ester comprised of butyrate and glycerol.

Moreover, the present inventors have found that acetic acid and butyric acid act in different, yet complementary pathways to improve gut homeostasis, gut bacterial ecology and Treg numbers and function. For example, without wishing to be bound by theory, the inventors believe that butyric acid acts through a Treg associated pathway that is distinct from that described for acetate and which includes enhanced TGFβ production. Further, acetic acid is believed to be particularly useful in modifying the effects of antigen presenting cells, particularly B cells, thereby modifying the frequency of autoimmune T effector cells.

The SCFAs may be provided in an individual requiring treatment by any number of means known to the skilled person. For example, in one embodiment, the SCFAs are provided in a pharmaceutical formulation according to the invention for oral, local or systemic administration, as further described herein. In a preferred embodiment, and as further described herein, the pharmaceutical formulation is adapted for delivery of the SCFAs to the large intestine, more particularly, the colon of the individual.

Alternatively, the SCFAs may be provided to the individual as part of the individuals' diet, whereby the SCFAs are provided for contact with the cells of the digestive tract upon digestion of a dietary agent in a desired region of the gastrointestinal tract. In a preferred embodiment, the dietary agent provides for release of the SCFAs in the colon, as further described herein.

Preferably, the combinations, pharmaceutical composition, and dietary agent of the invention are for use in the prevention and/or treatment of any disease which results in an increased autoimmune inflammatory response in one or more regions of the body. Therefore, the combinations, pharmaceutical composition, and dietary agent of the invention are for use in treating diseases associated with dysfunctional/ineffective regulatory T cell function, expanded autoreactive T effector cells, and/or B cell dysfunction. Diseases which may be prevented and/or treating in accordance with the present invention are autoimmune diseases, including, for example, an autoimmune disease selected from the group consisting of type 1 diabetes, psoriasis, rheumatoid arthritis, inflammatory bowel disease, caeliac disease, autoimmune hepatitis, myocarditis, lupus nephritis, primary biliary cirrhosis and multiple sclerosis.

Preferably, the combinations, pharmaceutical composition, and dietary agent of the invention have particular utility in the prevention and treatment of type 1 diabetes.

As used herein, "preventing", "prevention", "preventative" or "prophylactic" refers to keeping from occurring, or to hinder, defend from, or protect from the occurrence of a condition, disease, disorder, or phenotype, including an abnormality or symptom. An individual in need of prevention may be prone to developing an autoimmune disease. For example, preventing an autoimmune disease in accordance with the present invention includes preventing the onset of said disease in an individual identified as being at risk of developing the disease. An individual may be identified as being at risk either by way of genetic testing, analysis of environmental factors, family history or other factors.

An individual in need of treatment may be one diagnosed with, or at risk of developing, any one of the autoimmune diseases described herein. The term 'treatment', as used herein includes minimising the progression or delaying the progression of a disease. For example, the combinations of the present invention may be useful in preventing the onset of disease in an individual showing early signs of disease. As an example, in the context of type I diabetes, an individual with early signs of the disease may show signs of pancreatic islet damage, or have islet autoantibodies that are markers for pancreatic damage, but does not yet have abnormal glucose tolerance. Further progression of the disease may include abnormal glucose tolerance, but not yet requiring insulin treatment. The skilled person will appreciate that the combinations of the present invention are useful for the treatment of type I diabetes in any of these contexts.

In addition to primates, such as humans, a variety of other mammals can be treated according to the combinations, dietary agent and pharmaceutical composition of the present invention. For instance, mammals including, but not limited to, cows, sheep, goats, horses, dogs, cats, guinea pigs, rats or other bovine, ovine, equine, canine, feline, rodent or murine species can be treated according to the present invention.

### Pharmaceutical formulations

A pharmaceutical composition for use in treating or for use in delaying the progression of an autoimmune disease, wherein the composition includes a combination of butyric acid and acetic acid, esters or salts thereof and pharmaceutically acceptable excipients, wherein the butyric acid, acetic acid, esters or salts thereof are the active ingredients in the composition, and wherein the butyric acid and acetic acid are conjugated to a carrier in the form of a starch molecule.

The pharmaceutical compositions of the invention can enable delivery of the SCFAs butyric acid and acetic acid in the individual requiring treatment for an autoimmune disease.

It will be understood to those skilled in the art, that the pharmaceutical compositions described herein include the SCFAs butyric acid and acetic acid in the form of free fatty acids, esters or salts as conjugates to a carrier in the form of a starch molecule, such as acetylated or butyrylated starches.

For example, a combination according to the invention comprising two or more SCFAs for use in the treatment for an autoimmune disease may be administered to a person requiring treatment in a single pharmaceutical dosage form. The dosage form may comprise acetic acid and butyric acid, salts or esters thereof. Yet further, the dosage form may comprise all three of acetic acid, butyric acid and propionic acid, including salts or esters thereof.

The pharmaceutical compositions of the invention may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

The pharmaceutical compositions of the invention may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the SCFA active ingredients in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatine or acacia, and lubricating agents, for example magnesium stearate, stearic acid or talc.

### Delayed release oral dosage forms

The present inventors have surprisingly found that delivery of a high dosage combination of acetate and butyrate to the lower intestinal tract of a subject, impacts greatly on the development and progression of autoimmune disease, and in particular type 1 diabetes (T1D). The pharmaceutical formulations of the present invention , allow for a very high level of SCFA to be provided in the small or large intestine, including the colon, so as to contribute directly to improvements in gut homeostasis, gut bacterial ecology and Treg numbers and function.

Accordingly, in one embodiment, the pharmaceutical compositions of the invention are for use in treating or preventing an autoimmune disease in an individual in need thereof, wherein the treatment or prevention includes:
administering to the individual a pharmaceutical dosage form including a therapeutically effective amount of a combination of two or more short chain fatty acids, esters or salts thereof according to the invention, wherein the short chain fatty acids are selected from the group consisting of butyric acid and acetic acid and propionic acid;
wherein the pharmaceutical dosage form is adapted for release of the short chain fatty acids into the lower gastrointestinal tract of the individual;
thereby treating or preventing the autoimmune disease.

The skilled person will be familiar with methods for producing delayed release oral dosage forms which facilitate delivery of the active agents to a desired region of the gastrointestinal tract.

The term "delayed release," as used herein, refers to a delivery of SCFAs which is achieved by formulating the pharmaceutical composition comprising the SCFAs so that their release will be accomplished at some generally predictable location in the lower GI tract more distal to that which would have been accomplished had there been no alteration in the delivery of the SCFAs.

The term "gastrointestinal tract" or "GI tract," as used herein, relates to the alimentary canal, i.e., the musculo-membranous tube about thirty feet in length, extending from the mouth to the anus. The term "upper gastrointestinal tract," as used herein, means the buccal cavity, the pharynx, the esophagus, and the stomach. The term "lower gastrointestinal tract," as used herein, means the small intestine and the large intestine.

The term "small intestine," as used herein, means the part of the lower gastrointestinal tract consisting of the duodenum, the jejunum, and the ileum, i.e., that portion of the intestinal tract just distal to the duodenal sphincter of the fundus of the stomach and proximal to the large intestine.

The term "large intestine," as used herein, means the part of the lower gastrointestinal tract just distal to the small intestine, beginning with the cecum, including the ascending colon, the transverse colon, the descending colon, the sigmoid colon, and the rectum.

In certain embodiments of the present invention, it may be desirable to achieve delivery of the SCFAs (either as free acids, salts or esterified acids) to the small intestine or a particular segment thereof (e.g., the duodenum, jejunum or ileum). In still other instances, it may be desirable to deliver the SCFAs in a bolus amount to the small intestine.

In certain embodiments of the present invention, it may be desirable to achieve delivery of the SCFAs (either as free acids, salts or esterified acids) to the large intestine or a particular segment thereof (e.g., the ascending colon). In still other instances, it may be desirable to deliver the SCFAs in a bolus amount to the large intestine.

In one embodiment, the oral dosage form comprises an enteric coating which is resistant to degradation in the stomach, but which dissolves once the dosage form exits the stomach and enters the small intestine. In an alternative embodiment, the oral dosage form comprises an enteric coating which is resistant to degradation in the stomach and small intestine but dissolves once the dosage form arrives in the large intestine. The skilled person will be familiar with the use of enteric coating materials to control release of the active ingredient contained in the pharmaceutical dosage form such that the active ingredient is released in a specified location in the gastrointestinal tract.

The skilled person will appreciate that the ultimate site of and/or the rate of delivery in the small or large intestine can be satisfactorily controlled by one skilled in the art, by manipulating any one or more of the following:
(a) the type of coating, the type and level of excipients added to the coating and the concomitant desirable thickness and permeability (swelling properties) of the coating;
(b) the time dependent conditions of the coating itself and/or within the coated tablet, particle, bead, or granule;
(c) the pH dependent conditions of the coating itself and/or within the coated tablet, particle, bead, or granule;
(d) the dissolution rate of the coating;

A human or other mammal suffering from and requiring treatment for an autoimmune disease, can in certain embodiments of the present invention, be successfully treated by the delivery of SCFAs to the large intestine of said human or other mammal. The dosage forms described herein effect a release to the large intestine, and prohibit the undesired release of the SCFAs in the mouth, pharynx, esophagus, stomach, and/or small intestine, thereby preventing the degradation of the SCFAs before they release their intended site in the gastrointestinal tract.

Various means for targeting release of the SCFAs in the small or large intestine, including the colon are suitable for use in the present invention. Non-limiting examples of means for delivery to the large intestine include pH triggered delivery systems and time dependent delivery systems.

One embodiment of the present invention involves coating (or otherwise encapsulating) the SCFAs with a substance which is not broken down, by the gastrointestinal fluids to release the SCFAs until a specific desired point in the intestinal tract is reached. In one embodiment, delayed release of the pharmaceutical composition is achieved by coating the tablet, capsule, particles, or granules, of the SCFAs with a substance which is pH dependent, i.e,, broken down or dissolves at a pH which is generally present in the large intestine, but not present in the upper gastrointestinal tract (i.e., the mouth, buccal cavity, pharynx, esophagus, or stomach) or lower GI tract.

One embodiment of the present invention is delivered to the small or large intestine utilizing a pH dependent enteric coating material made from a partly methyl esterified methacrylic acid polymer. The oral dosage form can be in the form of an enteric coated compressed tablet made of granules or particles of active ingredient. Any enteric coating which is insoluble at a pH below 5.0 (i.e., that generally found in the mouth, pharynx, esophagus, stomach), but soluble between about pH 5.5 and about pH 7.5 (i.e., that present in the small and large intestine) can be used in the practice of the present invention. For example, when it is desired to effect delivery of the SCFAs to the large intestine, any enteric coating is suitable which is wholly- or partially-insoluble at a pH below 6.5 and soluble above pH 6.5.

The skilled person will appreciate that the pH varies along the digestive tract and will be able to determine a suitable enteric coating to ensure that the dosage form disintegrates and the active ingredients are released at an appropriate or desired location in the gastrointestinal tract.

Methacrylic acid copolymers which are suitable for use in coating the oral dosage forms and/or the granules, particles, or beads of active ingredient which can be employed in the method of treatment described herein, either alone or in combination with other coatings, are anionic carboxylic polymers. It is particularly preferred that the polymers are acrylic polymers, most preferably partly methyl-esterified methacrylic acid polymers, in which the ratio of anionic free carboxyl groups to ester groups is about 1:1.

A particularly suitable methacrylic acid copolymer is Eudragit L^{®}, particularly Eudragit L-30-D^{®} and Eudragit 100-55^{®}, manufactured by Rohm Pharma GmbH, Weiterstadt, West Germany. In Eudragit L-30-D^{®}, the ratio of free carboxyl groups to ester groups is approximately 1:1. Further, said copolymer is known to be insoluble in gastrointestinal fluids having a pH below 5.5, generally 1.5-5.5, i.e., that generally present in the fluid of upper gastrointestinal tract, but readily soluble at pH above 5.5, i.e., that generally present in the fluid of the lower gastrointestinal tract. Such copolymers are useful for enteric coatings intended to facilitate release of the active ingredients into the small intestine.

Alternative copolymers are Eudragit S^{®} and Eudragit FS30D^{®}, manufactured by Rohm Pharma GmbH and Co. KG, Darmstadt, Germany. Eudragit S^{®} differs from Eudragit L 30 D-55^{®}, only insofar as the ratio of free carboxyl groups to ester groups is approximately 1:2. Eudragit S^{®} is also, like Eudragit L 30 D-55^{®}, substantially insoluble at pH below 5.5, but unlike Eudragit L 30 D-55^{®}, is poorly soluble in GI fluids having a pH of 5.5-7.0, such as that present in small intestinal fluids. Eudragit S^{®} is soluble at pH 7.0 and above, i.e., that generally present in the terminal ileum and colon.

Eudragit S^{®} can also be used alone as a coating which would provide delivery of the SCFA ingredients beginning primarily at the large intestine (more distal than the terminal ileum) via a delayed-release mechanism. In addition, Eudragit S^{®}, being poorly soluble in intestinal fluids below pH 7.0, could be used in combination with Eudragit L 30 D-55^{®}, soluble in intestinal fluids above pH 5.5, in order to effect a delayed release composition which could be formulated to deliver the active ingredient at various segments of the intestinal tract; the more Eudragit L 30 D-55^{®} used, the more proximal release and delivery begins and the more Eudragit S^{®} used, the more distal release and delivery begins.

The coating can, and usually will, contain a plasticizer and possibly other coating excipients such as coloring agents, surfactant, talc, and/or magnesium stearate, many of which are well known in the coating art. In particular, anionic carboxylic acrylic polymers usually will contain 10-25% by weight of a plasticizer, especially triethyl citrate, tributyl citrate, acteyltriethyl citrate, dibutyl phthalate, diethyl phtbalate, polyethylene glycol, acetylated monoglycerides, propylene glycol, and triacetin.

Conventional coating techniques such as fluid-bed or pan coating are employed to apply the coating. Coating thickness must be sufficient to ensure that the oral dosage form remains essentially intact until the desired site of delivery in the small intestine is reached.

The oral dosage form may be in the form of a coated compressed tablet which contains particles or granules of the SCFAs, or of a soft or hard capsule (e.g., gelatine, starch, or hydroxypropylmethylcellulose), coated or uncoated, which contains beads or particles of the SCFAs, which themselves are enterically coated. In an embodiment of the invention the tablets are compressed and the tablet is enteric coated.

### Time dependent delivery systems and bacterial enzyme triggered systems

In another embodiment of the invention, delivery of the SCFAs to the large intestine is achieved through a time dependent delivery system. Given established transit times after gastric emptying, SCFA release (either as free acid or esterified acids) can be targeted to the various segments of the large intestine.

Approaches to time dependent delivery systems suitable for use in the present invention include, but are not limited to, such devices as the PulsincapTM (Scherer DDS, Strathclyde, U.K.), the Time ClockTM (Zambon Group, Milan, Italy), and SyncroDose TM (Penwest, Patterson, NY), as well as various coatings which degrade over time to release tablet contents such as hydroxypropylmethylcellulose, hydroxypropylcellulose, or any suitable hydrogel.

In one embodiment of the invention, delivery of the SCFAs to the large intestine is achieved through a bacterial enzyme triggered system. Oral dosage forms from which drug release is triggered by the action of bacterial enzymes in the colon are known in the art. Various approaches to bacterially-triggered delivery systems suitable for use in the present invention include disulfide polymers, glycosidic prodrugs, and polysaccharides as matrices/coating agents (Watts & Illum, 1997). Further approaches to bacterially-triggered delivery systems suitable for use are disclosed in Katsuma et al., 2004). In one embodiment of the invention, the colon-targeted delivery system CODES^{™} (Yamanouchi Pharma Technologies, Norman, Okla.) is used to deliver the SCFAs to the colon. This system comprises a tablet core containing SCFAs, and a saccharide, which tablet core is coated with an acid soluble material, such as Eudragit E^{®}, and then coated with an enteric coating, such as Eudragit L^{®}. The enteric coating protects the dosage form from degradation in the stomach, and is subsequently dissolved in the small intestine following gastric emptying. The acid-soluble coating protects against degradation as the dosage form travels through the small intestine. When the dosage form reaches the large intestine, local microflora ferment the saccharide (e.g., lactulose) in the tablet core into short chain fatty acids (such as isobutyrate, butyrate, isovalerate, valerate, isocaproate and caproate) which then dissolve the acid-soluble coating to release the core tablet contents in the colon.

Accordingly, the CODES system provides yet a further means of providing an increased amount of butyric acid to the colon of an individual in need thereof.

In a further embodiment, the dosage form also comprises a therapeutically effective amount of butyric acid, esters or salts thereof in the tablet core.

The skilled person will be familiar with alternative methods which may be employed for colon-targeted delivery of SCFAs including pressure dependent systems, CODES^{™} technology, microsponges, pectin and galactomannan coating, microbially triggered osmotic systems and lectins.

Formulations for oral use may also be presented as hard gelatin capsules wherein the SCFAs are mixed with an inert solid diluent, for example, calcium carbonate, calcium phosphate or kaolin, or as soft gelatin capsules wherein the SCFAs are mixed with water or an oil medium, for example peanut oil, liquid paraffin, or olive oil.

Aqueous suspensions may contain the SCFAs in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the SCFAs in a vegetable oil, for example arachis oil, olive oil, sesame oil or coconut oil, or in a mineral oil such as liquid paraffin. The oily suspensions may contain a thickening agent, for example beeswax, hard paraffin or acetyl alcohol. Sweetening agents such as those set forth above, and flavouring agents may be added to provide a palatable oral preparation. These may be preserved by the addition of an anti-oxidant such as ascorbic acid.

Dispersible powders and granules suitable for preparation of an aqueous suspension by the addition of water provide the SCFAs in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally- occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxy ethylene sorbitan monooleate. The emulsions may also contain sweetening and flavouring agents.

Syrups and elixirs may be formulated with sweetening agents, for example glycerol, propylene glycol, sorbitol or sucrose. They may also contain a demulcent, a preservative and flavoring and coloring agents.

### Rectal suppositories

The method of the present invention also contemplates the provision of SCFAs in the large intestine via a pharmaceutical dosage form which is provided as a rectal suppository. Accordingly, in a particular embodiment, the pharmaceutical compositions of the invention are formulated as suppositories for rectal administration of the SCFAs. These formulations can be prepared by mixing the SCFAs with a suitable non-irritating excipient which is solid at ordinary temperatures but liquid at the rectal temperature and will therefore melt in the rectum to release the drug. Such materials include cocoa butter and polyethylene glycols. Rectal administration may be used to eliminate entero-hepatic first pass effect in the gastro-intestinal tract related to oral administration of active agents. In yet a further embodiment, the rectal suppositories may include the SCFAs provided as esterified modified starches, wherein upon release of the modified starch in the rectum, the starch becomes available to the resident microbiota for digestion and consequent release of the SCFAs as metabolites of digestion.

### Injectable formulations

The present invention also involves injectable pharmaceutical formulations according to the invention for systemic delivery of the SCFAs. For example, the SCFAs may be directly injected into the bloodstream of the individual for whom treatment or prevention of an autoimmune disease is required. The injection may be adapted for intravenous or intraarterial injection. In an alternative embodiment, the injectable formulation may be adapted for subcutaneous injection, so as to facilitate either local administration, or a delayed release into the bloodstream.

The pharmaceutical compositions of invention may be in the form of a sterile injectable aqueous or oleagenous suspension. This suspension may be formulated according to the known art using those suitable dispersing or wetting agents and suspending agents which have been mentioned above. The pharmaceutical compositions may also be a sterile injectable solution or suspension in a non-toxic parenterally-acceptable diluent or solvent, for example as a solution in 1 ,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The pharmaceutical compositions of the invention may conveniently be presented in dosage unit form and may be prepared by any of the methods well known in the art of pharmacy. All methods include the step of bringing the SCFAs together with the pharmaceutically acceptable excipients which constitutes one or more accessory ingredients. In general, the pharmaceutical compositions are prepared by uniformly and intimately bringing the SCFAs into association with a liquid carrier or a finely divided solid carrier or both, and then, if necessary, shaping the product into the desired formulation.

The pharmaceutical compositions of the invention, may also be formulated in liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolisable lipid capable of forming liposomes can be used. The liposome formulation may contain stabilisers, preservatives, excipients and the like. The preferred lipids are the phospholipids and phosphatidyl cholines, both natural and synthetic. Methods to form liposomes are known in the art.

The pharmaceutical compositions of the invention may be included in a container, pack, or dispenser together with instructions for administration.

The term "pharmaceutically acceptable" as used herein means the carrier, diluent or excipient is not deleterious to the recipient thereof.

Unless otherwise indicated herein, the terms "composition" and "formulation" are used interchangeably.

The terms "administration of and or "administering" should be understood to mean providing to an individual in need of treatment.

### Dietary agent

The invention pertains to a dietary agent for delivery of a combination of short chain fatty acids into the large intestine of an individual, the agent including a carrier covalently bonded to the short chain fatty acids by a bond that is hydrolysable in the colon of an individual, to give free fatty acid, wherein the combination of short chain fatty acids is butyric acid and acetic acid, preferably wherein the combination further includes propionic acid, more preferably wherein the carrier is a carbohydrate selected from the group consisting of a starch, gum, oligosaccharide or pectin.

Acylated high-amylose starches are known. For example, such starches are described in US 5,840,860, and Annison et al., 2003. Specifically, acetylated high-amylose maize starch (HAMSA), butyrylated high-amylose maize starch (HAMSB) and propionate high-amylose maize starch (HAMSP) are known, as is high-amylose maize starch which does not comprise esterified fatty acid (HAMS). The use of these acylated starches individually is known, however, before now, utilising combinations of these starches has not previously been contemplated.

The present inventors have surprisingly found that delivery of a high dosage combination of acetate and butyrate through diet is a simple approach that impacts greatly on the development and progression of autoimmune disease, and in particular type 1 diabetes (T1D). Diet and bacterial metabolites represents a highly promising alternative to pharmaceutical approaches, to prevent or treat T1D and other autoimmune diseases.

The combinations, dietary agent and compositions of the present invention allow for a very high level of SCFA to be released in the lower colon, and significantly higher levels than those obtained through intake of dietary fibre alone. Specifically, because the acylated starches described herein are resistant to degradation in the small intestine of the individual, and have higher amounts of fatty acid than starches found in normal diets, the dietary agents described herein enable the provision of significantly higher doses of short chain fatty acids in the large intestine of an individual which can be administered or taken by the individual in a convenient and safe form.

Exemplary methods for preparing dietary agents for use in the method of the present invention are further described herein.

Dietary metabolites operate at many levels to correct defects in gut or immune homeostasis, limit number of autoreactive T cells, and prevent disease. One important feature of the present invention is that dietary delivery of high amounts of acetate, a natural product, was shown to achieve changes in the B cell molecular profile in the whole animal. Further, butyrate was found to protect against T1D through a Treg associated pathway, distinctive from that described for acetate. Accordingly, the particular combination of a high acetate and butyrate treatment represents an exciting and simple means for manipulating the cells of the immune system, not just cells present in the colon.

The intestinal microbiota responds rapidly to changes in diet (Faith et al., 2014, Zelante et al., 2013) and prolonged use of certain diets permits depletion of stressed and uncompetitive bacteria and emergence of new faster growing strains due to adaptive mutations. In summary, the present inventors have demonstrated that the approach to using dietary metabolites represent a novel and effect means for disruption of autoimmune pathogenesis.

For example, in one embodiment, each molecule of carrier includes an acetic acid and a butyric acid moiety. In a further embodiment, each molecule of carrier includes at least one acetic acid, at least one butyric acid and at least one propionic acid molecule.

The carrier molecule is a carbohydrate selected from the group consisting of a starch, gum, oligosaccharide or pectin. Preferably the carbohydrate is a starch.

In a preferred embodiment, the dietary agent of the present invention includes a starch molecule acylated with two different SCFAs. For example, the starch may be acylated with a plurality of both butyric acid and acetic acid moieties. In a further embodiment, the starch is acylated with a plurality of butyric, acetic and propionic acid moieties.

The present invention also contemplates various carrier molecules having various degrees of substitution. For example, where the carrier is a starch molecule, the invention contemplates degrees of substitution ranging from 0.05 to 1.0, preferably 0.1 to 0.8 and more preferably 0.5. A degree of substitution of 0.5 means that on average throughout the starch molecule, there is one short chain fatty acid moiety per 2 glucose molecules. The skilled person will appreciate that the presence of short chain fatty acid moieties will not necessarily be uniform along the length of the starch molecule, but represents the number of moieties on average. The skilled person will also appreciate that where two short chain fatty acid moieties are present on a single molecule of starch, a degree of substitution of 0.5 is indicative of roughly 1 short chain fatty acid of one type per 4 molecules, assuming there are equal amounts of each short chain fatty acid moiety on the starch molecule.

### Dosage

It will be appreciated that the dose of the short chain fatty acids may vary depending on the mode of administration of the SCFA of the invention, the form in which it is provided (e.g., as an oral dosage form, injection or dietary agent) and the intended site of action of the short chain fatty acids.

Preferably, wherein the present invention involves the administration of an oral dosage form for delivery of the combination of short chain fatty acids into the large intestine of an individual, the dose is approximately 0.01 mg/kg to 100 mg/kg per day, preferably 0.1 mg/kg to 100 mg/kg per day, more preferably 1 mg/kg to 50 mg/kg. In a most preferred embodiment, the daily dose of any one of acetic acid, butyric acid or propionic acid is 2 mg/kg to 10 mg/kg, including 3, 4, 5, 6, 7, 8, and 9 mg/kg.

In circumstances where the dietary agent according to the invention for is used for delivery of the combination of short chain fatty acids into the large intestine, the skilled person will appreciate that the amount of agent or diet to be consumed will vary depending on the composition of the diet and the proportion of each short chain fatty acid incorporated into the carrier molecules included in the dietary agent.

In one embodiment, the dietary agent comprising a combination of two short chain fatty acids bound to a single carrier molecule, and the carrier is a starch having a degree of substitution of 0.4 (i.e., 1 short chain fatty acid approximately every 2.5 molecules), the dosage will be approximately 1 g of starch to 40 g of starch per day for a 50 kg individual, preferably 1 g of starch per day to 10 g of starch per day (i.e. 0.02 g to 0.2 g/kg per day). More preferably, the dose will be 2 g to 8 g per day (0.04 g/kg to 0.16 g/kg per day). More preferably the dose if approximately 3.75 g of the starch molecule per day for a 50 kg individual (or 0.075 g/kg/day). This corresponds to approximately 250 mg to 300 mg of each of the short chain fatty acids, assuming there are equal proportions of these in the starch molecule.

### Administration

In one embodiment, the SCFAs are provided in the individual for release in the large intestine of the individual, for contacting the cells of the large intestine with the SCFAs. This can be accomplished by a number of means, including by an enteric coated dosage form for oral administration, which is formulated for release of the SCFAs in the colon of the individual. Alternatively, the SCFAs can be provided in a pharmaceutical dosage form for rectal administration.

The pharmaceutical compositions of the invention may be administered by any suitable means, for example, orally, such as in the form of tablets, capsules, granules or powders; sublingually; buccally; parenterally, such as by subcutaneous, intravenous, intramuscular, intraperitoneal or intracisternal injection or infusion techniques (e.g., as sterile injectable aqueous or non-aqueous solutions or suspensions); nasally such as by inhalation spray; topically, such as in the form of a cream or ointment; or rectally such as in the form of suppositories or enemas; in dosage unit formulations containing non-toxic, pharmaceutically acceptable vehicles or diluents. They may, for example, be administered in a form suitable for immediate release or extended release, for example, by the use of devices such as subcutaneous implants, encapsulated spheroids or osmotic pumps.

In an alternative embodiment, the dietary agent of the invention is used in the administration of the SCFAs.

The skilled person will appreciate that any number of combinations may be utilised for providing the SCFAs according of the invention to the individual in need (i.e., combinations of pharmaceutical dosage forms, a combination of a dietary agent and a pharmaceutical dosage form or a combination of dietary agents).

### Combination therapy

The pharmaceutical formulations or dietary agents described herein may be used, in combination with methods for providing high levels of SCFAs to the large intestine of an individual requiring treatment or prevention of an autoimmune disease.

### Examples

### Example 1: Animal studies

### Experimental methods

### Animals and Models

The NOD/Lt (NOD), C57BL/6 and NOD.8.3 mice were derived from Monash Animal Research Platform, Melbourne Australia. Gpr43-/- mice (Maslowski, et al., 2009) and the MyD88-/- mice (obtained from Shizuo Akira), both on a C57BL/6 background were backcrossed >10 times to the NOD background. GF NOD mice were derived from Germ Free Unit (Walter and Eliza Hall Institute of Medical Research). NOD.FoxP3-GFP mice (NOD/ShiLt-Tg(FoxP3-EGFP/cre)1cJbs/J, obtained from The Jackson Laboratory, USA).

To standardise microbiota, prior to beginning diets mice from multiple litters were mixed and randomly allocated to groups. The purified diets used were based on a balanced modification of the AIN93-G diet as described previously (Bajka et al., 2006). Mice were fed for 3-5 weeks starting at 3, 5 or 10 weeks of age. SCFAs in faeces, blood and caecal content were analysed as previously described in (Bajka et al., 2006). Diabetes was monitored as previously described (Mariño et al., 2009).

All experimental procedures involving mice were carried out according to protocols approved by the relevant Animal Ethics Committee of Monash University, Melbourne, Australia.

### Animal diets

Control (LAMS), and acetylated (HAMSA), proprionylated (HAMSP) and butyrylated (HAMSB) starch diets contained the following ingredients:

### components as g/kg

| **Ingredient** | **LAMS** | **15% HAMSA** | **15% HAMSP** | **15% HAMSB** |
|---|---|---|---|---|
| Maize starch - 3401C | 529.5 | 379.5 | 379.5 | 379.5 |
| **HAMSA** | 0 | **150** | 0 | 0 |
| **HAMSP** | 0 | 0 | **150** | 0 |
| **HAMSB** | 0 | 0 | 0 | **150** |
| Casein | 200 | 200 | 200 | 200 |
| Sucrose | 100 | 100 | 100 | 100 |
| Sunflower Seed Oil | 70 | 70 | 70 | 70 |
| alpha cellulose | 50 | 50 | 50 | 50 |
| Mineral Mix AIN 93G | 35 | 35 | 35 | 35 |
| Vitamin Mix AIN 93VX | 10 | 10 | 10 | 10 |
| L-Cystine | 3 | 3 | 3 | 3 |
| Choline bitartrate | 2.5 | 2.5 | 2.5 | 2.5 |
| **Total** | **1000** | **1000** | **1000** | **1000** |

### Flow Cytometry And Lipopolysaccharide and Cytokine Measurement

Immunophenotypic analysis of mononuclear cells used the following mAbs: CD3e (145-2C11), CD4 (RM4-5), CD25 (PC61), CD8α (Ly2), CD44 (IM7), CD45R/B220 (RA3-6B2), IgM (11/41), CD45RB (16A), MHC II (I-Ak )(ABk) (10-3.6), MHC I (H- 2Kd), CD86 (B7-2) (GL1), CD80 (B7-1) (16-10A1), CD62L (MEL-14), CD103 (2E7), FoxP3 (FJK-16 s). Isotype controls included IgG1, λ; IgG; IgG2a, κ. Intracellular proteins used: IL-10 (JES5-16E3) as described in Mariño et al., 2012, HELIOS (22F6) and FoxP3 were detected as manufacturer's transcription factor staining protocol (eBioscience, Biolegend). All Abs from BD Bioscience, eBioscience or Biolegend). The stained samples were analysed using BD LSRII flow cytometers with FACSDiva software (BD Biosciences) and FlowJo software version 9.3.2 (Tomy Digital Biology). Spleen and PLN T cells were stained with PE-labelled IGRP206-214 (H-2K(d), VYLKTNVFL) tetramer, PE-labelled IAg7/BDC2.5mi (AHHPIWARMDA) tetramer. PE-labelled TUM (H-2K(d), KYQAVTTTL) tetramer and PE-labelled IAg7/human CLIP 87-101 (PVSKMRMATPLLMQA) tetramer were used as negative control in all experiments (provided by NIH Tetramer Facility, Atlanta, USA) with peptides from Mimotopes PTY LTD, Australia.

TNFα was measured by ELISA using BD OptEIA Kit (BD Biosciences), IL-21 by ELISA Ready-SET-Go! Kit (eBioscience) and IL-22 and TGFβ by biotinylated antibodies (Biolegend). Plasma lipopolysaccharides (LPS) was measured by ToxinSensorTM Chromogenic LAL Endotoxin Assay Kit (GenScript, USA Inc.) according to the manufacturer's manual.

### Bacteria DNA Sequencing and Bioinformatics

Bacterial genomic DNA from feces was extracted using QIAamp DNA stool mini kit (QIAGEN). DNA samples were amplified targeting the V1-V3 region of bacterial 16S rRNA gene using forward primer 5' AGAGTTTGATCCTGG 3'; and a reverse primer, 5'TTACCGCGGCTGCT 3' and sequenced using Roche 454 GS FLX+ sequencer.

Bioinformatics analysis was performed with Quantitative Insights into Microbial Ecology (QIIME) software. Chimeric sequences were detected and removed using the Pintail algorithm (Ashelford et al., 2005) and de-noised and error-corrected with Acacia (Bragg et al., 2012). OTUs were picked at 97% sequence identity using the uclust algorithm in QIIME. Taxonomies were assigned in QIIME using BLAST against the Greengenes database (DeSantis et al., 2006). The EzTaxon database was used to additionally compare representative OTU sequences with a database of culturable strains (Chun et al., 2007). Network was visualized in Calypso (http://cgenome.net/calypso/).

### Lamina Propria Treg cell Isolation, Adoptive Transfers

Colonic lamina propria lymphocytes were prepared as described previously (Arpaia et al., 2013). For adoptive transfer into NOD/SCID mice, NOD mice were fed various diets for 10 weeks, starting at 5 weeks of age. At 15 weeks, mice were culled and total T cells were isolated from spleen, peripheral lymph nodes using a Miltenyi Biotec Pan T Cell Isolation Kit II with a Miltenyi Biotec LS MACS separation column with ≥ 95% purity, and intravenously injected into 8 week-old NOD/SCID mice fed with NP diets and monitored for diabetes development. For analysis of Treg conversion CD3+B220-Foxp3- T cells were sorted using Influx sorter (> 95% purity) and transferred via i.v into NP-,HAMS-, HAMSA- and HAMSB-fed NOD/SCID mice. For the adoptive transfer of NOD.8.3 CD8+T cells, lymphocytes from spleen, PLN and MLN of NOD.8.3 mice were purified using the MACS CD8α+ T cells Isolation Kit (Miltenyi Biotech). 5×10⁶ CFSE labelled CD8+ T cells were injected intravenously into NOD recipients previously fed NP, HAMS, HAMSA or HAMSB for 2 weeks. Mice were continued on the same diet after 4 days post-transfer when splenic, PLN and MLN lymphocytes were harvested and analysed for CD8+ T cell proliferation by CFSE dilution.

### Histopathology

Pancreatic tissue was processed and stained using standard procedures. For insulitis scoring, pancreata sections were taken at 3 concentrations (100 µm apart). At least 100 islets were scored from 5 to 15 mice. Islets were graded according to the following system: Grade 0 - no indication of insulitis, Grade 1 - <25% infiltration, Grade 2 - 25-50% infiltration, Grade 3 - 50-75% infiltration, and Grade 4 - >75% infiltration.

### Gut microbiota oral gavage

Faecal and caecal contents were collected and resuspended in deoxygenated PBS at a ratio of 100 µg of material to 1ml of PBS. The mix was then homogenised, spun down at 1000 rpm for 5 min and the supernatant was used for gavages. Pregnant GF NOD mice and their pups were gavaged with 200µl of faecal and caecal mix from 8-10 week old HAMS-, HAMSA- and HAMSB-fed NOD donors. Mothers received two oral gavage of the mix at E18 of pregnancy and two weeks after giving birth. Pups received two oral gavage of the mix at weaning (20-26 day old) within 24 hrs. Then the pups were followed for disease incidence.

### Chromatin Immunoprecipitation (ChIP)

Chromatin immunoprecipitation (ChIP) was performed as described in (Thorburn et al., 2015). Briefly, sorted CD4+CD25- (> 95% purity) T cells were fixed in 0.6 % paraformaldehyde, washed with PBS then lysed in NP-40 lysis buffer (0.5 % NP-40, 10 mM Tris-HCL at pH 7.4, 10 mM NaCl, 10 mM MgCl2 and protease inhibitors) followed by SDS lysis buffer (1 % SDS, 10 mM EDTA, 50 mM Tris-HCl at pH 8.1 and protease inhibitors). DNA was sonicated for 30 cycles, 20sec on, 30sec off at 4°C using Bioruptor Next Gen (Diagenode). Sonicated chromatin product was diluted in 1% Triton X-100, 20 mM Tris-HCl at pH 8.0, 150 mM NaCl, 2 mM EDTA. ChIP was performed with either IgG control, anti-histone H3, anti-acetyl-histone H3 (Lys9) or anti-hyperacetylated histone H4 (Penta; K5, 8, 12, 16 and 20 acetylation) antibodies (Millipore, USA). Chromatin was isolated with protein A/G-Sepharose and washed with low salt buffer (0.1 % SDS, 1 % Triton X-100, 20 mM Tris-HCl at pH 8.1, 150 mM NaCl, and 2 mM EDTA), high salt buffer (0.1 % SDS, 1 % Triton X-100, 20 mM Tris-HCl at pH 8.1, 500 mM NaCl, and 2 mM EDTA) and diluted in LiCl buffer (0.5 % NP-40, 0.5 % deoxycholate, 10 mM Tris-HCl at pH 8.1, 1 mM EDTA and 0.25 M LiCl). DNA was recovered in elution buffer (1 % SDS and 100 mM NaHCO3), de-cross-linked by high salt treatment (200 mM NaCl) at 65°C and treated with proteinase K (40 µg/ml proteinase K, 10 mM EDTA, 40 mM Tris-HCl at pH 8.1) at 50 °C. DNA was isolated for qPCR using primers specific for Foxp3 promoter (F CTG AGG TTT GGA GCA GAA GGA, R GAG GCA GGT AGA GAC AGC ATT G). Fold increase in acetylation is presented relative to H3.

### Real time PCR Analysis and Treg cell Single-cell Expression Analysis

RNA from the colon was extracted and converted to cDNA using Bioline's Tetro cDNA synthesis kit, using oligo (dT)18 primers to amplify mRNA. qPCR was performed using Bioneer's Accupower 2x Greenstar qPCR master mix on Biorad's Cfx384 real time system. All expression was standardized to the housekeeping gene β-actin. CD4+CD45RBlowCD25+Treg cells from pooled PLN (n=10 mice) were sorted individually directly into a 96 well qPCR plate (Influx cell sorter instrument, BDBiosciences). Single-cell PCR was performed using The Life Technology Single-cell to CT kit according to manufacturer's instructions. Single-cell expression analysis was performed using a Biomark instrument (Fluidigm) as described previously (Polo et al., 2012). Results are expressed as Log2Ex=-(Cq [gene]-LOD). If Log2Ex value is negative, Log2Ex=0.

### RNA-seq

RNA extraction was done using RNeasy Qiagen kit. cRNA was hybridized to whole Mouse Genome Arrays using RNAseq sequencing by Illumina 100 - base HT mode sequencing chemistry in fragment end read format, barcoded samples per Illumina HiSeq 1500 lane. Briefly, RNA samples from 4 diets × 4 replicates were sequencing ~20 million reads per sample, with single-end sequencing.

**Table 1 (a) Differentially expressed transcripts in splenic IgM+B220+ B cells from of 15 week-old HAMSA- vs HAMS-fed female NOD mice, HAMSB- vs HAMS-fed female NOD mice and HAMSA- vs HAMSB-fed female NOD mice. Mean fold change in gene expression. A FDR cut-off of q<0.05 was used to define differentially expressed genes between groups. (b) List of oligo(dT)18 primers used to amplify mRNA for RT-PCR expression analysis.**

| | **Fold change** | | | **FDR** | | |
|---|---|---|---|---|---|---|
| **Genes** | **H_v_HA** | **H_v_HB** | **HA_v_HB** | **H_v_HA** | **H_v_HB** | **HA_v_HB** |
| ***SCAND1*** | **1.038765285** | **-0.761971898** | **-1.800737183** | **0.009681928** | **0.073246183** | **4.63E-09** |
| ***BCL7c*** | **0.534614945** | **-0.589566465** | **-1.12418141** | **0.106615615** | **0.032634043** | **3.11E-07** |
| ***SIK1*** | **-1.206305407** | **-2.688991855** | **-1.482686447** | **0.005951783** | **1.68E-16** | **0.00013211** |
| ***DDIT4*** | **-1.03054346** | **-2.239445709** | **-1.208902249** | **0.003461034** | **2.64E-17** | **0.000137753** |
| ***Nudc*** | **0.025389948** | **-0.758008984** | **-0.783398932** | **0.964870448** | **0.000186472** | **0.000291791** |
| ***DUSP1*** | **1.010796812** | **- 2.256518864** | **- 1.245722052** | **0.010466454** | **9.86E-16** | **0.000291791** |
| ***Hspalb*** | **-0.896237485** | **-4.701855414** | **-3.805617929** | **0.624228295** | **2.82E-06** | **0.000453239** |
| ***Hspa1a*** | **-0.737357947** | **-4.241367961** | **-3.504010014** | **0.705195043** | **2.77E-05** | **0.00211719** |
| ***Bpgm*** | **1.736110063** | **0.266462255** | **1.469647808** | **0.000635759** | **0.839821929** | **0.003713543** |
| ***PLAUR*** | **0.677592143** | **1.256048653** | **-0.57845651** | **0.001150789** | **5.91E-16** | **0.005879226** |
| ***slfn8*** | **-0.264876794** | **0.343844109** | **0.608720902** | **0.486096908** | **0.284249773** | **0.009762771** |
| ***Pydc3*** | **-0.590878226** | **0.368394563** | **0.959272789** | **0.289572828** | **0.591586016** | **0.013182284** |
| ***GNB3*** | **-2.550184011** | **1.425253743** | **3.975437755** | **0.374989511** | **0.397708401** | **0.022071431** |
| ***PEX11A*** | **-0.006217573** | **-1.433380409** | **-1.427162836** | **0.998049711** | **0.012472856** | **0.027801206** |
| ***Pde2a*** | **-0.023928691** | **0.480204947** | **0.504133637** | **0.964225683** | **0.036431625** | **0.034039725** |
| ***Ube2I6*** | **1.420254968** | **0.37743697** | **-1.042817998** | **0.008379746** | **0.740024467** | **0.080822966** |
| ***TMCC2*** | **5.082558393** | **3.895050929** | **1,187507464** | **0.000635759** | **0.108766891** | **0.130414757** |
| ***CD163*** | **2.237908576** | **0.99071786** | **-1.247190716** | **0.005951783** | **0.415842724** | **0.184827324** |
| ***IL10ra*** | **-0.597534123** | **-1.01103722** | **-0.413503097** | **0.035189002** | **4.44E-07** | **0.185764333** |
| ***TRIM10*** | **4.813601067** | **3.130643978** | **-1.682957089** | **0.002041285** | **0.045608569** | **0.317759899** |
| ***Cpa1*** | **-5.799112877** | **-8.672790674** | **-2.873677797** | **0.035189002** | **0.000233834** | **0.328908459** |
| ***Cpb1*** | **-5.09036293** | **-8.137973982** | **-3.047611052** | **0.175199751** | **0.005176466** | **0.332962482** |
| ***Crem*** | **- 1.618612279** | **2.091671886** | **0.473059608** | **0.000476181** | **2.31E-09** | **0.383081947** |
| ***NR4A1*** | **0.965821305** | **1.272320876** | **0.306499571** | **0.000984664** | **4.47E-08** | **0.399552621** |
| ***DUSPS*** | **-1.142883517** | **-1.524582305** | **-0.381698788** | **0.002041285** | **2.50E-07** | **0.407065508** |
| ***Gda*** | **2.237344604** | **1.574899514** | **- 0.662445091** | **0.001047466** | **0.022788387** | **0.415059631** |
| ***Ank1*** | **2.531364692** | **1.839304695** | **-0.692059997** | **0.002297301** | **0.032013499** | **0.486732105** |
| ***SLC4A1*** | **4.720822999** | **3.626282992** | **-1.094540007** | **0.001850047** | **0.009079279** | **0.505335049** |
| ***SPTB*** | **3.799103389** | **2.827882079** | **-0.971221309** | **0.004350384** | **0.030111291** | **0.513047904** |
| ***SPTA1*** | **4.401310873** | **3.304921413** | **-1.09638946** | **0.005951783** | **0.033209623** | **0.525327496** |
| ***Epb4.2*** | **4.760517378** | **3.664031859** | **-1.096485519** | **0.008635805** | **0.033197498** | **0.564152371** |
| ***Cd69*** | **-0.782923669** | **-0.936994531** | **-0.154070862** | **0.005726972** | **4.14E-05** | **0.676359646** |
| ***Id3*** | **0.762776968** | **0.740373181** | **-0.022403787** | **0.005951783** | **0.002482835** | **0.960273075** |

| **b** | **Primer name** | **Forward sequence** | **Reverse sequence** |
|---|---|---|---|
| | ***Actb*** | **ACC AGA GGC ATA CAG GGA CA** | **CTA AGG CCA ACC GTG AAA AG** |
| | ***Ocln*** | **TCC GTG AGG CCT TTT GAA** | **GGT GCA TAA TGA TTG GGT TTG** |
| | ***Tjp1*** | **GCC AGA TAT CTA TCA GAT TGC AAA A** | **GAT GCC AGA GCT ACG ATG G** |
| | ***Cdh1*** | **ACC GGA AGT GAC TCG AAA TGA TGT** | **CTT CAG AAC CAC TGC CCT CGT AAT** |
| | ***Muc2*** | **TCC TGA CCA AGA GCG AAC AC** | **ACA GCA CGA CAG TCT TCA GG** |
| | **87-1** | **TTC CCA GCA ATG ACA GAC AG** | **CCA TGT CCA AGG CTC ATT CT** |
| | **B7-2** | **CAC GAG CTT TGA CAG GAA CA** | **TTA GGT TTC GGG TGA CCT TG** |
| | ***Prdm1*** | **CAG AGA GCA GAG CAC CTC AG** | **ACA TGT AGC AAG GCC ATC AA** |
| | ***82m*** | **CCT TCA GCA AGG ACT GGT CT** | **TGT CTC GAT CCC AGT AGA CG** |

### Statistical Analysis

Statistical significance for comparing two independent groups was determined by calculating P-values using non-parametric Mann-Whitney U test (GraphPad Prism software, La Jolla, CA, USA). For all diet data, the following comparisons were made: (1) NP vs. HAMS, (2) HAMS vs. HAMSA, (3) HAMS vs. HAMSB, (4) NP vs HAMSA, (5) NP vs HAMSB. Diabetes incidence studies were graphed as Kaplan-Meier survival plots and analysed using the Mantel-Cox log-rank test with 2-degrees of freedom. ****P<0.0001, ***P<0.001, **P<0.01, *P<0.05.

### RESULTS

### Gut Microbial Metabolites Correlate With Protection Against Diabetes

Peripheral blood concentrations of the SCFA acetate in SPF NOD.MyD88-/- mice were highly elevated (2-3 fold), compared to age- and gender-matched NOD mice (Figure 1a). This was also consistent with high concentrations of acetate in the feces of SPF NOD.MyD88-/- mice (Figure 9b), suggesting a positive relationship between SCFA-producing bacteria and protection. Butyrate was also increased in peripheral blood (Figure 1a), although this metabolite is barely detectable under normal conditions. High concentrations of butyrate in hepatic portal vein blood was found in SPF NOD.MyD88-/- mice (50-100 µM) compared to SPF NOD mice (5-20 µM) (Figure 1b). Acetate concentrations in the hepatic portal vein blood support the results observed in peripheral blood.

To determine if microbiota-derived SCFAs modify disease incidence, disease progression in normal NOD mice housed under GF conditions was examined. Diabetes penetrance was higher in the cohort of GF NOD mice compared to SPF NOD mice (Figure 1c), and this was associated with markedly decreased concentrations of butyrate in feces (Figure 9B). Although fecal acetate concentrations do not change significantly between SPF and GF NOD mice, much higher concentrations of fecal acetate and butyrate were found in SPF NOD.MyD88-/- mice compared to GF (Figure 9b).

Insulitis in the pancreatic islets appears at ~5 weeks of age in NOD mice and overt diabetes occurs between 15 to 30 weeks of age in 60-80% of females but only in 20-30% of males. Consistently, it was found that young female NOD mice had significantly lower concentrations of both acetate and butyrate in the peripheral blood compared to age-matched male NOD mice (Figure 1d). Gender differences in fecal SCFA concentrations were not seen in C57/BL6 mice (Figure 9c), suggesting insufficient bacterial production of SCFAs is a particular phenomenon to the female NOD mouse.

To determine whether oral administration of acetate (to increase its concentration in the circulation) could modulate disease progression, acetate was added to the drinking water of NOD female mice. Starting from 5 weeks of age, acetate treatment significantly delayed the development of diabetes (incidence 40% vs 70% in controls at age 30 weeks) (Figure 1e). By 10 weeks of age, 5 weeks after starting treatment, acetate-treated NOD mice had more pancreatic islets without immune cell infiltration, and fewer islets with high-grade infiltration (insulitis scores of 3 or 4) (Figure 1f).

### Increasing SCFAs by High Acetate- and Butyrate-yielding Diets Protect Against T1D

Oral administration of SCFAs produces a rapid and transient rise in their circulating concentrations but does not model their sustained absorption from the colon through bacterial fermentation of dietary fiber (Topping & Clifton, 2001 and Illman et al., 1988). In addition to the combined effects of SCFAs derived from fiber, an important question is the role of individual SCFAs in T1D risk. Therefore, specialized high amylose maize starch (HAMS) diets were used, either acetylated (HAMSA) or butyrylated (HAMSB). Acylated starches (HAMS) are largely resistant to small intestinal amylolysis and pass to the colon where bacteria release their specific incorporated SCFAs. HAMS is then fermented with the production of the normal range of SCFAs. These starches have proven to be powerful tools for assessing the effects of specific SCFAs (i.e. acetate versus butyrate) on intestinal biology (Fukuda et al., 2011, Clarke et al., 2008, Bajka et al., 2010).

Upon feeding female NOD mice with HAMSA or HAMSB diets for 5 weeks, concentrations of acetate or butyrate were markedly increased in feces, cecal digesta, hepatic portal blood and peripheral venous blood compared to control HAMS-fed NOD mice (Figure 2a). These results demonstrate the efficacious power of high acetate and butyrate-yielding diets targeting colonic bacteria to produce acetate and butyrate, respectively, and how they reach peripheral circulation in high concentrations. Acetate is regarded as an end-product of anaerobic fermentation, however butyrate-producing bacteria such as *Faecalibacterium prausnitzii* can be net users of acetate. This may account for slightly higher concentrations of butyrate in HAMSA fed mice. Likewise higher gut propionate concentrations in both HAMSA and HAMSB fed mice suggest that high delivery of acetate or butyrate fed back to bacterial production of propionate (Figure 2a). Mice on either diet had similar body weights to HAMS-fed or nonpurified (NP) diet-fed mice (Figure 9d). The diets did not significantly alter propionate concentrations in portal or peripheral blood.

In NOD mice the immuno-inflammatory events associated with progression from insulitis to clinical diabetes occur between 5-15 weeks of age (Mariño, et al. 2008, Ayyavoo et al., 2013). Autoantibodies to antigens such as insulin can appear as early as 5 weeks indicating that breakdown of immune tolerance occurs well before signs of overt diabetes.

To determine if specialized high SCFA-yielding diets administered relatively late could affect development of T1D, female NOD mice were fed for 5 weeks starting at 10 weeks of age with HAMS, HAMSA or HAMSB and mice were then returned to the control NP diet and monitored for diabetes incidence. By 30 weeks of age, female NOD mice continuously fed the NP diet developed diabetes at the expected high frequency (Figure 2b). The HAMS diet did not significantly delay diabetes compared to NP-fed female NOD mice. In contrast, 71% of HAMSA-fed and 62% of HAMSB-fed female NOD mice were protected from T1D (Figure 2b). Representative histology and insulitis scoring showed that, compared to 15 week-old HAMS-fed NOD mice, age-matched HAMSA- and HAMSB-fed female NOD mice had a higher number of islets with no infiltration (Grade 0), a difference that was maintained up to 30 weeks of age (Figure 2c).

NOD mice were fed with a combination of HAMSA and HAMSB (15% +15%) exactly as performed with the other diets. Figure 2d shows that using this combo diet, 100% of NOD mice were protected from diabetes through to 28 weeks.

### HAMSA Feeding in Reduces Autoimmune T Cell Frequencies

Pathogenesis of T1D involves the killing of islet β-cells by autoreactive T cells. Strikingly, both diets but particularly acetate-yielding HAMSA markedly decreased the frequency of splenic diabetogenic CD8+ T cells that recognize the islet-specific antigen glucose-6-phosphatase catalytic subunit related protein (IGRP), and CD4+ cells carrying the diabetogenic T-cell receptor BDC2.5 (Figure 3a, b). Frequency of IGRP-reactive tetramer+ CD8+ T cells were reduced from ~1% in control HAMS fed NOD mice to 0.2% in HAMSA-fed mice (Figure 3a) and BDC2.5-reactive tetramer+ CD4+ T cells were reduced 2 fold (Figure 2b).

NOD 8.3 mice develop diabetes rapidly with an incidence of ~100% by 10 weeks of age. Interestingly, NOD.8.3 mice fed the HAMSA diet showed a significant delay in develop of diabetes (Fig 3c). Percentage of IGPR-reactive T cells was markedly reduced in HAMSA- but not HAMSB-fed NOD8.3 mice (Fig 3d).

### Acetate Effects on B Cells Protects Against T1D in NOD Mice

SCFAs might impair autoimmune responses by affecting other cells types, such as APCs that support autoreactive T cell expansion. In order to examine this, NOD.8.3 mice fed with different diets. The colony of NOD.8.3 mice develop diabetes rapidly with an incidence of ~100% by 10 weeks of age, since they express TCRαβ transgenes derived from a CD8+ T cell clone NY8.3 that recognises IGRP61. NOD.8.3 mice fed with HAMSA (but not HAMSB) diet showed a significantly delayed development of diabetes (Figure 3c). Numbers of IGRP-reactive T cells was markedly reduced in HAMSA-, but not HAMSB-fed NOD8.3 mice (Figure 3d). Frequency of Treg cells in HAMSA-fed NOD8.3 mice was similar to that observed in HAMSB- and HAMS-fed NOD8.3 mice (data not shown) suggestive of another mechanism. Antigen-presenting B cells play an important role in CD8+ expansion in NOD mice. Transfer of NOD8.3 T cells into NOD.µMT^{(-/-}) or NOD/SCID mice, which lack B cells, fails to efficiently transfer diabetes, but transfer is restored by prior engraftment with B cells (Mariño et al., 2012, Ziegler et al., 2013). In previous studies, B cells were found to promote disease in NOD mice, by directly presenting islet antigens to CD4+ and CD8+ T cells, and suppressing Treg cell function (Bajka et al., 2010, Lagger et al., 2002, Yamaguchi et al., 2010). HAMSA-fed NOD mice showed reduced frequency and number of IgM+ B220+ B cells in the spleen and Peyer's patches (Figure 4a). Strikingly, IgM+B220+ B cells from spleen and PLN from HAMSA fed (but not HAMSB-fed) NOD mice showed markedly reduced expression of MHC I by flow cytometry, as well as CD86, co-stimulatory molecules for APCs (Figure 4b, 10a). No changes were observed in CD40, MHC II or CD80 expression (Figure 10b). To substantiate these findings, B cells from mice fed the different diets were interrogated using gene microarrays.

Changes in total B cells at the protein level were validated at the level of gene expression for *CD80*/*B7.1, CD86*/*B7.2, B2M, PRDM1 (Blimp1)* and *MYD88* (Fig. 10c). β2-microglobulin and MyD88 are essential for MHC I expression and Blimp1 for regulating B cell proliferation and differentiation. Interestingly, both HAMSA and HAMSB reduced CD86 expression in IL12-producing mature marginal zone (MZB) B cells (Fig. 4c), a subset implicated in corruption of immune tolerance. The changes in expression in B cells for the above gene transcripts suggested that acetate or butyrate diets may be having a profound effect on global gene transcription. RNA sequencing was performed on 95% pure sorted splenic B cells isolated from 15 week-old mice fed for 5 weeks with NP, HAMS, HAMSA or HAMSB diets. In a multidimensional scaling analysis, IgM+ B220+ B cells from HAMSA-fed mice are further apart from the control samples (HAMS or NP) (Fig. 4d), indicating an increase in variability that supports the increased expression. Indeed 208 genes were differentially expressed between HAMSA and HAMSB, compared to NP diet. 14 genes are involved in important B cell functions, such as antigen presentation, BCR signalling, cell metabolism and activation of cytotoxic T cells (Fig. 4e and Table 1a). Increased dietary SCFAs down-regulated heat shock protein genes such as *Hspa1a* and *Hspa1b*, and increased urine levels have been linked with progressive renal failure in children with T1D (Yilmaz et al., 2016). Similarly, both HAMSA and HAMSB down-regulated expression of *II10ra.* In infection models, blockade of the IL10 receptor significantly diminishes plasma B cells and precipitates infection. SCFAs inhibit HDAC activity and maintain histone acetylation. Increased expression of *Gnb3* on B cells was found, a gene linked with reduced expression of HDAC3. Mice deficient in HDAC 1 and 2 show disrupted B cell development, survival and activation. There was markedly reduced expression of HDAC3 transcripts in mature B cell subsets (MZB and FOB) from mice fed HAMSA (Fig. 4f), which presumably relates the significant changes in gene expression we observed. This correlated with reduced expression of MHC I and CD86, explaining the reduced capacity of B cells to cross-present autoantigen to CD8+ T cells.

To test whether HAMSA or HAMSB affected APC capacity to support autoreactive T cell proliferation *in vivo* in NOD mice, purified CFSE-labelled NOD8.3 CD8+ T cells were transferred into 8-10 week-old NOD mice that had been fed HAMS, HAMSA or HAMSB. Thus the response of NOD8.3 CD8+ T cells should relate mainly to dietary effects on cell types such as APCs, rather than directly on the responding NOD8.3 T cells. 3-4 days posttransfer proliferation of transferred islet-specific NOD8.3 CD8+ T cells was analysed and it was found that they divided extensively in the PLN of NOD mice fed the NP diet, and to a lesser (but not significant) degree in the PLN of NOD mice fed the HAMS diet (Figure 4g). Strikingly, when NOD mice were fed HAMSA, autoreactive NOD8.3 CD8+ T cells failed to proliferate in the PLN. Proliferation of NOD.8.3 T cells in the PLN was not reduced by HAMSB feeding. T cell proliferation was specific to the PLN (where islet autoantigens drain and APC-T cell activation occurs), since NOD.8.3 T cells did not proliferate in the MLN of any of the diet groups including NP (Figure 10d). Thus dietary acetate affects B cell numbers, their phenotype and appears to affect their capacity to expand autoreactive T cells *in vivo.*

### Butyrate modulates Treg numbers and function in lymphoid tissues in NOD mice

SCFAs modulate Treg cells, although effects have been demonstrated in the gut their effects on other primary cells important for the development of T1D was unclear. The T cell compartment of 15 week-old female NOD mice fed with HAMSA and HAMSB diets was examined and it was found that both diets expanded a population of CD4+FoxP3+ Treg cells in the colon that expressed the gut homing activation marker CD103 (Figure 11a), similar to that reported for C57BL/6 mice. The feeding of HAMSB to NOD mice resulted in very high levels of butyrate in hepatic portal vein. Compared to HAMS-fed NOD mice, both acetate- and butyrate-yielding diets increased 2 fold the frequency and number of splenic Treg cells (Figure 5a). In the PLN and MLN a modest increase in frequence was observed in the absolute number of Treg cells (Figure 11b).

>95% purified splenic T cells from 15 week-old female NOD mice fed the different diets for 10 weeks were transferred into female NOD/SCID mice devoid of T and B cells. Use of NOD/SCID recipients ensured that any effects of diets on T cells could be attributed entirely from the conditioned donor T cells, and that effects could be examined in isolation from acetate/butyrate effects on gut homeostasis or other systems. T cells from NP-fed NOD mice rapidly transferred diabetes in all recipients with an accelerated onset after transfer (Figure 5b). In marked contrast, 94% of NOD/SCID recipients that received T cells from HAMSB-fed NOD mice were completely protected from diabetes for 20 weeks or more after transfer (Figure 5b). T cells from HAMSA-fed NOD mice failed to fully protect (30% diabetes-free mice at 20 weeks after transfer), despite the effect of HAMSA on frequency of T effector cells (Figure 3a-d).

In order to determine whether HAMSB-conditioned Treg cells mediated protection observed in NOD/SCID mice, total Foxp3- T cells from NOD.FoxP3-GFP mice were adoptively transferred. Recipient NOD/SCID mice were fed with different diets 2 weeks prior to adoptive transfer, and these mice remained on the same diets and were monitored for disease incidence. Three weeks post-transfer, CD4+ T cells were analysed for the expression of Foxp3, IL-10 and HELIOS. Remarkably, only the HAMSB-fed NOD/SCID mice showed significant frequency and number of CD4+ T cells that expressed Foxp3, IL-10 and HELIOS (Figure 5c, d).

Acetylation of the histones at the FOXP3 locus of peripheral Treg cell (CD4+CD25-) precursors has previously been associated with enhanced Treg function (Thorburn et al., 2015). Given the high concentrations of acetate and butyrate observed in HAMSA- and HAMSB-fed NOD mice, particularly in the hepatic portal vein (Figure 2a), high acetate- or butyrate-yielding diets were examined for their influence on histone modifications at the FOXP3 locus, particularly in peripheral Treg cells *in vivo.* The HAMSB diet, but not HAMSA, markedly increased H3K9 acetylation and H4 pentacetylation at the Foxp3 promoter (Figure 5e) in splenic T cells. To examine whether histone acetylation in peripheral Treg cell precursors was associated with increased Foxp3 expression in Treg cells, single cell transcriptome analysis of individual sorted CD4+CD45RBlowCD25+ T cells (Treg gate) was performed. Only HAMSB resulted in increased Foxp3 transcript expression compared to HAMS-fed NOD mice (Figure 5f). In addition, Treg cells of HAMSB-fed NOD mice had increased expression of gene transcripts including *Gata3*, *Gitr* and *Sell* (CD62L) (Figure 5g), which are important for Treg cell activation, function and migration.

### Metabolite-Sensing GPCRs GPR43 and GPR109a Play a Minor Role in NOD Diabetes

The principal metabolite-sensing GPCR for acetate and butyrate is GPR43, which is expressed by diverse immune cell types including activated macrophages, B cells, and Treg cells. To determine if immunomodulatory effects of acetate and butyrate were mediated through this GPCR, C57.Gpr43-/- mice were backcrossed 13 generations to the NOD background (*NOD. Gpr43-*/*-)* (Figure 12a). There were trends (but no significant differences) in disease incidence between *NOD.Gpr43+*/*+* versus *NOD.Gpr43-*/*-* mice. By 20 weeks 70% of *NOD.Gpr43-*/*-* mice had developed diabetes similar to *NOD.Gpr43+*/*+* littermates on the same diet (Figure 6a). However, *NOD.Gpr43-*/*-* mice displayed more islet inflammation (insulitis grade 1 to 4) and showed fewer islets with no infiltration (<20%) (Figure 6b). This suggested only a minor role for GPR43 in protection from β-cell islet destruction. However the HAMSA diet only partially delayed diabetes progression in *NOD. Gpr43-*/*-* mice, and these mice had less infiltrated islets compared to NP-fed *NOD.Gpr43-*/*-* mice (Figure 6a, b). A cellular phenotypic analysis showed *NOD.Gpr43-*/*-* mice contained significantly reduced numbers of Treg cells and higher numbers of IgM+B220+ B cells in the spleen and PLN (Figure 6c, d), compared to *NOD.Gpr43+*/*+* littermates. HAMSA increased Treg cells, and decreased autoreactive T cells in *NOD.Gpr43+*/*+* mice, but not in *NOD.Gpr43-*/*-* littermates (Figure 6 c-e). Acetylated starch diets were still able to deliver acetate in quantity, as measured in the feces of 15 week-old female *NOD. Gpr43-*/*-* mice (Figure 12b-d). Disease incidence in a CRISPR generated *NOD.Gpr109a-*/*-* mouse line showed that 80% of *NOD.Gpr 109a-*/*-* mice progressed to diabetes at a similar rate to WT NOD mice (Figure 13a).

### Both Acetate and Butyrate Correct Defective Gut Epithelial Integrity in NOD Mice

Autoimmune diabetes in humans and mice is associated with 'gut leakiness' and low expression of the tight junction protein occluding. In female NOD mice, there was evidence that the intestinal wall was compromised in its capacity to form an effective barrier, based on decreased expression of occludin and other tight junction markers, relative to their expression in C57BL/6 (but not Balb/c mice) (Figure 7a). In contrast, male NOD mice, with a lower incidence of diabetes, had higher expression of tight junction markers relative to C57BL/6 and Balb/c mice (Figure 7a). Feeding of high acetate- and butyrate-yielding diets significantly increased the expression of occludin in the colon of NOD mice compared to HAMS-fed NOD controls (Figure 7b). Consistent with the role of SCFAs in preserving epithelial gut integrity and dampening tissue inflammation, HAMSA and particularly HAMSB diets resulted in a significant reduction in serum lipopolysaccharide (LPS) concentrations similar to those in C57BL/6 mice (Figure 7c).

Increased circulating pro-inflammatory cytokines are associated with progression to T1D. Both HAMSA and HAMSB diets promoted a switch in cytokine profile from proto anti-inflammatory (Fig. 7d, e). Serum concentrations of TNFα and IL-21, two cytokines required for development of diabetes in NOD mice, were markedly decreased in either delayed HAMS-fed NOD mice or protected HAMSA- and HAMSB fed NOD mice, similar to what was observed in protected NOD.MyD88-/- mice (Figure 7d). IL-22, a gut homeostasis-related cytokine that maintains gut mucosal barrier integrity, was significantly elevated in HAMSA-fed (Figure 7e) and, to a lesser extent, in HAMSB-fed NOD mice compared to control HAMS-fed NOD mice and diabetogenic NP-fed NOD mice.

### Acetate-enriched Diet Leads to Beneficial Changes in Gut Microbial Ecology

The present study examined whether the protective effects of HAMSA and HAMSB diets were attributable at all to changes in the abundance and/or diversity of certain bacteria in the gut. GF NOD mice were re-colonised with gut bacteria from 15 week-old NOD mice fed on NP, HAMS, HAMSA or HAMSB (as shown in Figure 2b) by gavage with a mix of feces and cecal contents. The microbiota-reconstituted NOD mice were then all placed on the same NP diet and monitored over time for development of diabetes. GF NOD mice re-colonized with HAMS-shaped microbiota did not show a difference in disease incidence compared to SPF HAMS-fed donor mice (Figure 8a). However, GF NOD mice that received a microbiota shaped by HAMSA diet showed a marked protection against diabetes, with 80% of GF female NOD mice diabetes free for over 30 weeks (Figure 8a). GF NOD mice re-colonized with a HAMSB-shaped microbiota progressed rapidly to diabetes, suggesting that the HAMSB diet mediates its effects through direct supply of butyrate and not alteration of the intestinal microbiota. Fecal bacteria analysis showed that both acetate- and butyrate-yielding diets (compared to control HAMS diet) resulted in significant changes in fecal microbial composition in either 15 week-old donor NOD mice fed with diets, and in age-matched re-colonized GF NOD mice (Figure 8b, 13b). Despite both HAMSA- and HAMSB-fed donor mice showing increased abundance of *Bacteroides* (Figure 8c), no differences were found after the re-colonization of GF NOD mice (Figure 13c). *Bacteroides* is an acetate/butyrate-producing anaerobic bacteria and in humans and mice has been associated with induction of higher Treg cell numbers. HAMSA alone significantly decreased the abundance of *Lactobacillus* and *Parabacteroides* in donor and in re-colonized GF NOD mice (Figure 8c, 13c). GF NOD mice that received HAMSA-shaped microbiota showed increased abundance of genus Clostridium (Figure 13c). GF NOD mice reconstituted with a HAMSA-shaped microbiota had significantly higher concentrations of acetate in portal hepatic blood and cecal contents (Figure 8d) compared to GF NOD mice reconstituted with a HAMS-shaped microbiota. GF NOD mice re-colonised with HAMSA-shaped microbiota showed increased CD4⁺Foxp3⁺ Treg cells in the PLN (Figure 8e), supporting the role of acetate in Treg biology. Thus HAMSA diet-mediated protection occurred through changes across the whole bacterial community, and outgrowth of acetate producing bacteria.

SPF HAMSA-fed NOD mice showed significantly decreased fecal concentrations of glutamate, glutamine, leucine and isoleucine compared to HAMS-fed NOD mice (Figure 8f). Glutamate is a key bacterial metabolite that plays an important role in metabolic processes and stress responses, mainly through actions of glutamate decarboxylase (GAD) a known pancreatic islet autoantigen. Reduced glutamate was found in 15 week-old NOD mice fed the HAMSA diet and in the protected GF NOD mice recolonised with HAMSA-shaped microbiota (Figure 8f). Isoforms of the leucine family were increased in HAMSA-fed NOD mice (Figure 13f).

### DISCUSSION

Specialised acetylated or butyrylated starch diets in combination showed a remarkable protection from autoimmune diabetes in the NOD mouse. The effects of acetate and butyrate were only partially overlapping, indicative of different mechanisms. Acetate was particularly effective at decreasing frequencies of autoreactive T cells, while butyrate was superior for increasing the function of Treg cells. The metabolite approach reported here corrected virtually all of the defects in the NOD mouse that contribute to disease- gut microbiota composition and gut integrity, inflammatory cytokines such as IL-21 and TNFα, defective Treg biology, and abundance of autoreactive effector T cells. These findings highlight the profound influence of diet, gut bacteria and their metabolites on immune responses. Indeed, numerous immune parameters, including frequencies of important immune subsets (Tregs, B cells and effector T), as well as their gene expression profile, were shown to be manipulated simply by dietary/bacterial metabolites.

HAMSA-delivered acetate had a marked effect on autoimmune T effector cell frequencies. This could be explained by effects of acetate (and less so butyrate) on APCs, particularly B cells. B cells play an important role in the transition from insulitis to clinical diabetes, through their interactions with specific islet antigen-reactive T cells. B cells that lack sufficient co-stimulatory molecules can be tolerogenic. The important feature of our study was that delivery of high amounts of acetate, a natural product, could achieve changes in the B cell molecular profile in the whole animal. The reason that acetate but not butyrate was effective in altering peripheral B cell phenotype may relate to the much higher levels of acetate that could be delivered to the peripheral circulation, as butyrate is typically metabolized in the liver. However, acetate could also act through distinct pathways, for instance its ability to down-regulate *Hdac3* transcription. Down-regulation of co-stimulatory molecules and MHC I on B cells in HAMSA-fed NOD mice correlated with low frequency of IGRP+CD8+ (Fig. 3a, b) and greatly diminished expansion of autoreactive NOD8.3 cells *in vivo* (Fig. 3d). Autoreactive T cell numbers in T1D patients may be dramatically reduced by simple delivery of high acetate-yielding diets. Reduced frequencies of autoreactive T cells, measured through tetramer monitoring of T1D patients, could provide a much quicker indication that metabolite diets are having a desired effect, rather than waiting years for clinical outcomes.

HAMSB-conditioned T cells (including Treg cells) almost entirely protected against T1D upon transfer to NOD/SCID recipient mice. Thus, butyrate protects against T1D through a Treg associated pathway, distinctive from that described above for acetate. This likely relates to increased transcription of key Treg cell genes, and the ability of butyrate (but not acetate) to inhibit the enzymatic activity of HDACs. A previous study showed that a genetic defect in NOD mice uniquely affects a specific Treg cell subpopulation localizing in the PLN, rendering them defective in suppressive activity (Buhlmann et al., 1995). Therefore, butyrate may have corrected the impaired suppressive function of PLN (or peripheral) Treg cells. HAMSB enhanced TGFβ production, important for Treg cell function. In addition, acetate and butyrate may affect Treg cells indirectly, through suppression of inflammatory cytokines and LPS in blood and tissues, or controlling Treg versus Th17 differentiation. IL-21 in particular is critical during the development of T1D. It induces proliferation of B cells and autoreactive cytotoxic CD8+ cells in NOD mice84-86, and negatively regulates Treg cell differentiation and activity.

The impact of diet and metabolites on T1D pathogenesis may relate to the proximity of the pancreas or PLNs to the gut. T1D may be particularly amenable to a dietary metabolite approach, because the pancreas and PLN have direct lymphatic connections with the gut and may be influenced by the high concentrations of acetate or butyrate in the hepatic portal vein and possibly the peritoneal cavity. It is also conceivable that immune cells recirculate between a high acetate or butyrate environment (i.e. the colon) to the PLN, spleen and elsewhere.

Shaping of the gut microbiota to one of high SCFA production may be a strategy to prevent or treat human disease. SCFAs are one of the main metabolites of commensal bacteria, particularly members of the *Bacteroides* genus. The specialized diets used here expanded the numbers of *Bacteroides* species and led to greater numbers of Treg cells. A greatly increased proportion of *Clostridium* genus in GF recolonized NOD mice was observed, indicating that these are likely to be important for expansion of Treg cell numbers in the colon and periphery (Fig. 8e). In one study, abundance of bacterial species that either produce acetate, such as *Bifidobacterium adolescentis,* or *Clostridium cluster XIVa* or *IV* species (which produce butyrate from acetate), correlated inversely with a number of β-cell autoantibody specificities (de Goffau et al., 2013). We do not exclude the possibility that the gut microbiota selected by the HAMSA diet promoted other beneficial metabolite pathways. For example, microbes that produce tryptophan metabolites that bind the aryl hydrocarbon receptor (AhR) enhance gut homeostasis and Treg biology (Thorburn et al., 2014, Zelante et al., 2013, Li et al., 2011). Most efforts to correct dysbiosis have relied on probiotics, but perhaps the best means to correct an adverse microbiota composition is through use of diets as illustrated here. The intestinal microbiota responds rapidly to changes in diet (David et al., 2014, Kau et al., 2011) and prolonged use of certain diets permits depletion of stressed and uncompetitive bacteria and emergence of new faster growing strains due to adaptive mutations (Zhu and Ye, 2003, Zhu and Yang 2003).

At the molecular level, protection from diabetes likely involves engagement of metabolite-sensing GPCRs such as GPR43, as well as inhibition of HDACs. This allows for immediate signaling events through GPCRs, as well as changes in gene transcription. GPR43 facilitated at least part of the protective effect of the high acetate-yielding HAMSA diet. Numbers of Treg cells in several tissues, including PLN were markedly reduced in *NOD.Gpr43-*/*-* mice. GPR43 also affected the ability of acetate diet to limit autoreactive effector T cell frequencies. It is conceivable that GPR43 acts cooperatively with epigenetic mechanisms. For instance, GPR43 signalling may facilitate acetate entry to the cell. The other major role for GPR43 is enhancement of gut epithelial barrier function. It achieves this through activation of the inflammasome pathway in epithelial cells and production of the pro-gut homeostasis cytokine IL-18. Thus it is likely that GPR43 operates at several levels in diet-mediated protection from T1D. The other major pathway whereby metabolites influence immune responses is HDAC inhibition. Acetate delivery *in vivo* markedly diminished *Hdac3* transcript expression in B cells. This would presumably lead to a phenotype similar to that of HDAC3 deficiency in select cells, or HDAC enzymatic inhibition with chemical inhibitors or butyrate, which previous studies have shown to be anti-inflammatory. In DCs, broad HDAC inhibition leads to marked down-regulation of CD40, CD80 and CD8696 similar to what we observed here in B cells with acetate diet. Our studies also showed that HAMSB/butyrate enhanced histone H3 acetylation in the promoter region of the Foxp3 locus, in splenic Treg cells.

### Example 2: Preparation of acylated starches containing two or more fatty acids (large scale)

### Method:

1) DMSO (24 L) was heated to above 80° C. with an immersion heater in a metal vessel under constant stirring.
2) The heater was removed and maize starch (440 g) was added slowly to the stirring DMSO through a domestic sieve to ensure uniform dispersement (to avoid clumping). The mixture was stirred constantly for 1 h by which time all the starch had dissolved to leave a clear, viscous solution.
3) 1-MID (80 mL) was added and one, two or three anhydrides selected from acetic - 85 mL, propionic - 132 mL and butyric 170 mL.
4) After 4 h incubation the excess anhydride was decomposed by addition of 3 L of water and the reaction mixture was poured into 2 vols of ethanol.
5) The precipitated Acylated Starch product was washed with ethanol (80% v/v) several times to remove the DMSO and other reactants and dried at 40° C. in a hot air room.
6) A control starch went through a sham procedure in which no 1-MID or anhydride was added to the starch.
7) The starches were ground to a fine powder and analysed.

### Results

The DS of each of the Acylated Starch products produced above were similar. It will be understood that where only one anhydride is used at step 3, the acylated starch contains only 1 species of short chain fatty acid moiety. Where 2 anhydrides are used (for example, acetic acid and butyric acid), the acylated starch will contain a mixture of both acetic and butyric ester moieties. Where 3 anhydrides are used, the acylated starch will contain a mixture of all three fatty acid moieties.

### Example 3: Preparation of SCFA enriched diet

Large amounts (~500 g) of acylated starch, were prepared by the large scale procedure detailed in Example 2 above.

A combination diet containing both acetylated and butyrylated starch was prepared and contained the following ingredients:
- casein (200 g/kg)
- methionine (1.5 g/kg)
- sucrose (50 g/kg)
- starch (251.5 g/kg)
- corn oil (100 g/kg)
- mineral mix (35 g/kg)
- vitamin mix (10 g/kg)
- choline tartrate (2 g/kg)
- cellulose (50 g/kg)
- acetylated starch (150 g/kg)
- butyrylated starch (150 g/kg)

The percentage of acylated starch in the above diet is 30% (15%:15% acetylated: butyrylated starch).

An alternative combination diet can be prepared containing:

| ***components as g*/*kg*** | |
|---|---|
| **Ingredient** | **15%/15%** |
| Maize starch - 3401C | 229.5 |
| **Acetylated starch** | 150 |
| **Butyrylated starch** | 150 |
| Casein | 200 |
| Sucrose | 100 |
| Sunflower Seed Oil | 70 |
| alpha cellulose | 50 |
| Mineral Mix AIN 93G | 35 |
| Vitamin Mix AIN 93VX | 10 |
| L-Cystine | 3 |
| Choline bitartrate | 2.5 |
| **Total** | **1000** |

The diets can be cold extruded into pellets, dried and stored at low temperature prior to use. Alternatively, the diets can be prepared as powders, for inclusion into foods as described herein.

It will be appreciated that a similar diet can be prepared using 300 g of an acylated starch containing both acetate and butyrate moieties (rather than 150 g of a starch acylated with only one species of short chain fatty acid).

### Example 4: Food product containing acetylated and butyrylated starches

The acylated starches described herein can be used in powder form as supplements in various foods. For example:
A. Recipe for rapid dough containing acetylated and butyrylated starch:
   - 80 parts flour
   - 10 parts acetylated starch
   - 10 parts butyrylated starch
   - 2 parts fat
   - 2 parts salt
   - 1 part improver
   - 2.5 parts yeast
B. Other foods that can be supplemented using the acylated starches described herein:
   The powders of acylated starches described above can also be added to a range of hot foods including pasta sauces, risotto, gravy, soups and porridge or to cold foods including milk, cereal, chocolate/vanilla custard, pudding and juices.

### References

Annison, G., Illman, R.J. & Topping, D.L. Acetylated, propionylated or butyrylated starches raise large bowel short-chain fatty acids preferentially when fed to rats. The Journal of Nutrition 133, 3523-3528 (2003).
Arpaia, N., et al. Metabolites produced by commensal bacteria promote peripheral regulatory T-cell generation. Nature 504, 451-455 (2013).
Ashelford et al., Appl Environ Microbiol 71: 7724-7736 (2005)
Ayyavoo, A., et al., First-born children have reduced insulin sensitivity and higher daytime blood pressure compared to later-born children. The Journal of Clinical Endocrinology and Metabolism 98, 1248-1253 (2013).
Bajka et al., Br J Nutr. 2006 Aug;96(2):276-82 (2006)
Bajka, B.H., et al. Butyrylated starch increases large bowel butyrate levels and lowers colonic smooth muscle contractility in rats. Nutrition Research 30, 427-434 (2010).
Bragg et al., Nature Methods 9, 425-426 (2012)
Buhlmann, J.E., et al. In the absence of a CD40 signal, B cells are tolerogenic. Immunity 2, 645-653 (1995).
Chun et al., Int J Syst Evol Microbiol 57:2259-2261 (2007)
Clarke, J.M., et al., Effects of high-amylose maize starch and butyrylated high-amylose maize starch on azoxymethane-induced intestinal cancer in rats. Carcinogenesis 29, 2190-2194 (2008).
David, L.A., et al. Diet rapidly and reproducibly alters the human gut microbiome. Nature 505, 559-563 (2014).
de Goffau, M.C., et al. Fecal microbiota composition differs between children with beta-cell autoimmunity and those without. Diabetes 62, 1238-1244 (2013).
Faith, J.J., et al., Identifying gut microbe-host phenotype relationships using combinatorial communities in gnotobiotic mice. Science Translational Medicine 6, 220ra211 (2014).
Feehily, C. & Karatzas, K.A. Role of glutamate metabolism in bacterial responses towards acid and other stresses. J Appl Microbiol 114, 11-24 (2013).
Fukuda, S., et al. Bifidobacteria can protect from enteropathogenic infection through production of acetate. Nature 469, 543-547 (2011).
Illman, R.J., et al. Hypocholesterolaemic effects of dietary propionate: studies in whole animals and perfused rat liver. Annals of Nutrition & Metabolism 32, 95-107 (1988).
Katsuma et al., Journal of Pharm. Sci. 93(5): 1287-99 (2004).
Kau, A.L., Ahern, P.P., Griffin, N.W., Goodman, A.L. & Gordon, J.I. Human nutrition, the gut microbiome and the immune system. Nature 474, 327-336 (2011).
Lagger, G., et al. Essential function of histone deacetylase 1 in proliferation control and CDK inhibitor repression. Embo J 21, 2672-2681 (2002).
Li, Y., et al. Exogenous stimuli maintain intraepithelial lymphocytes via aryl hydrocarbon receptor activation. Cell 147, 629-640 (2011).
Macia, L., et al. Metabolite-sensing receptors GPR43 and GPR109A facilitate dietary fibre-induced gut homeostasis through regulation of the inflammasome. Nat Commun 6, 6734 (2015).
Mariño, E., et al. Marginal-zone B-cells of nonobese diabetic mice expand with diabetes onset, invade the pancreatic lymph nodes, and present autoantigen to diabetogenic T-cells. Diabetes 57, 395-404 (2008).
Mariño, E., et al. CD4(+)CD25(+) T-Cells Control Autoimmunity in the Absence of B-Cells. Diabetes 58, 1568-1577 (2009).
Mariño, E., et al., B-Cell Cross-Presentation of Autologous Antigen Precipitates Diabetes. Diabetes 61, 2893-2905 (2012).
Maslowski, K.M., et al. Regulation of inflammatory responses by gut microbiota and chemoattractant receptor GPR43. Nature 461, 1282-1286 (2009).
Polo, J.M., et al. A molecular roadmap of reprogramming somatic cells into iPS cells. Cell 151, 1617-1632 (2012).
Thorburn, A.N., Macia, L. & Mackay, C.R. Diet, metabolites, and "western-lifestyle" inflammatory diseases. Immunity 40, 833-842 (2014).
Thorburn, A.N., et al. Evidence that asthma is a developmental origin disease influenced by maternal diet and bacterial metabolites. Nature Communications 6, 7320 (2015).
Topping, D.L. & Clifton, P.M. Short-chain fatty acids and human colonic function: roles of resistant starch and nonstarch polysaccharides. Physiol Rev 81, 1031-1064 (2001).
Watts, P.J., & Illum, L., Drug Dev. and Indus. Pharm., 23(9): 893-917 (1997).
Yamaguchi, T., et al. Histone deacetylases 1 and 2 act in concert to promote the G1-to-S progression. Genes Dev 24, 455-469 (2010).
Yilmaz, A., et al. Higher urine heat shock protein 70/creatinine ratio in type 1 diabetes mellitus. Renal Failure 38, 404-410 (2016).
Zelante, T., et al. Tryptophan catabolites from microbiota engage aryl hydrocarbon receptor and balance mucosal reactivity via interleukin-22. Immunity 39, 372-385 (2013).
Zhu, C. & Ye, Q. Selection of acetate-tolerant mutants from Escherichia coli DH5alpha and the metabolic properties of mutant DA19. Wei Sheng Wu Xue Bao 43, 460-465 (2003).
Zhu, Y. & Yang, S.T. Adaptation of Clostridium tyrobutyricum for enhanced tolerance to butyric acid in a fibrous-bed bioreactor. Biotechnol Prog 19, 365-372 (2003).
Ziegler, A.I., et al. The CD19 signalling molecule is elevated in NOD mice and controls type 1 diabetes development. Diabetologia 56, 2659-2668 (2013).

## Claims

1. A therapeutically effective amount of a combination of two or more short chain fatty acids, esters or salts thereof, for use in treating an autoimmune disease or for delaying or preventing the progression of an autoimmune disease in an individual,
wherein the combination of short chain fatty acids is butyric acid and acetic acid, preferably wherein the combination further includes propionic acid, and wherein the short chain fatty acids are conjugated to a carrier in the form of a starch molecule.

2. The therapeutically effective amount of a combination of two or more short chain fatty acids, esters or salts thereof for the use of claim 1, wherein the treating the autoimmune disease includes reducing or treating inflammation in the individual, preferably wherein reducing or treating inflammation includes reducing the proportion of one or more pro-inflammatory cytokines in the individual or increasing the proportion of one or more anti-inflammatory cytokines in the individual.

3. A therapeutically effective amount of a combination of two or more short chain fatty acids, esters or salts thereof, for use in preventing or delaying the onset of autoimmune disease in an individual,
wherein the combination of short chain fatty acids, esters or salts thereof, is butyric acid and acetic acid or esters or salts thereof, preferably wherein the combination further includes propionic acid, and wherein the short chain fatty acids are conjugated to a carrier in the form of a starch molecule.

4. The therapeutically effective amount of a combination of two or more short chain fatty acids, esters or salts thereof for the use of claim 3, wherein the individual is determined to be at risk of developing an autoimmune disease, preferably wherein the individual is determined to have autoantibodies or inflammatory markers associated with a risk of developing an autoimmune disease.

5. The therapeutically effective amount of a combination of two or more short chain fatty acids, esters or salts thereof for the use of any one of the preceding claims, wherein the autoimmune disease is selected from the group consisting of: type 1 diabetes, psoriasis, rheumatoid arthritis, inflammatory bowel disease, caeliac disease, autoimmune hepatitis, myocarditis, lupus nephritis, multiple sclerosis or primary biliary cirrhosis, preferably wherein the autoimmune disease is type 1 diabetes.

6. The therapeutically effective amount of a combination of two or more short chain fatty acids, esters or salts thereof for the use of any one of the preceding claims wherein the autoimmune disease is type 1 diabetes or psoriasis.

7. The therapeutically effective amount of a combination of short chain fatty acids, esters or salts thereof, for the use of any one of the preceding claims, wherein the short chain fatty acids are in the form of a combination of starch molecules covalently bonded to acetic acid and of starch molecules covalently bonded to butyric acid.

8. The therapeutically effective amount of a combination of short chain fatty acids, esters or salts thereof, for the use of any one of claims 1 to 6, wherein each starch molecule is conjugated to at least one acetic acid molecule and to at least one butyric acid molecule.

9. A dietary agent for delivery of a combination of short chain fatty acids into the large intestine of an individual, the agent including a starch covalently bonded to the short chain fatty acids by a bond that is hydrolysable in the colon of an individual, to give free fatty acid, wherein the combination of short chain fatty acids is butyric acid and acetic acid, preferably wherein the combination further includes propionic acid.

10. The dietary agent of claim 9, wherein the dietary agent comprises a combination of starch molecules covalently bonded to acetic acid and of starch molecules covalently bonded to butyric acid.

11. The dietary agent of claim 9, wherein the dietary agent comprises starch molecules that are covalently bonded to at least one butyric acid and to at least one acetic molecule.

12. A pharmaceutical composition for use in treating or for use in delaying the progression of an autoimmune disease, wherein the composition includes a combination of butyric acid and acetic acid, esters or salts thereof and pharmaceutically acceptable excipients, wherein the butyric acid, acetic acid, esters or salts thereof are the active ingredients in the composition, and wherein the butyric acid and acetic acid are conjugated to a carrier in the form of a starch molecule.

13. The pharmaceutical composition for use according to claim 12, wherein the composition comprises a combination of starch molecules covalently bonded to acetic acid and of starch molecules covalently bonded to butyric acid

14. The pharmaceutical composition for use according to claim 12, wherein the composition comprises a starch molecule conjugated to at least one acetic acid molecule and to at least one butyric acid molecule.

15. The dietary agent for use of any one of claims 9 to 11, or the pharmaceutical composition for use of claim 12 to 14, wherein the agent or composition are for treating or delaying the progression of type 1 diabetes or psoriasis.

## Patentansprüche

1. Therapeutisch wirksame Menge einer Kombination aus zwei oder mehr kurzkettigen Fettsäuren, Estern oder Salzen davon zur Verwendung bei einem Behandeln einer Autoimmunerkrankung oder zum Verzögern oder Verhindern des Fortschreitens einer Autoimmunerkrankung bei einem Individuum,
wobei die Kombination aus kurzkettigen Fettsäuren Buttersäure und Essigsäure ist, vorzugsweise wobei die Kombination ferner Propionsäure einschließt, und wobei die kurzkettigen Fettsäuren an eine Trägersubstanz in Form eines Stärkemoleküls konjugiert sind.

2. Therapeutisch wirksame Menge einer Kombination aus zwei oder mehreren kurzkettigen Fettsäuren, Estern oder Salzen davon zur Verwendung nach Anspruch 1, wobei das Behandeln der Autoimmunerkrankung das Verringern oder Behandeln einer Entzündung bei dem Individuum einschließt, vorzugsweise wobei das Verringern oder Behandeln der Entzündung das Verringern des Anteils eines oder mehrerer entzündungsfördernder Zytokine bei dem Individuum oder ein Erhöhen des Anteils eines oder mehrerer entzündungshemmender Zytokine bei dem Individuum einschließt.

3. Therapeutisch wirksame Menge einer Kombination aus zwei oder mehr kurzkettigen Fettsäuren, Estern oder Salzen davon zur Verwendung bei dem Vorbeugen oder Verzögern des Ausbruchs einer Autoimmunerkrankung bei einem Individuum,
wobei die Kombination aus kurzkettigen Fettsäuren, Estern oder Salzen davon Buttersäure und Essigsäure oder Ester oder Salze davon ist, vorzugsweise wobei die Kombination ferner Propionsäure einschließt und wobei die kurzkettigen Fettsäuren an eine Trägersubstanz in Form eines Stärkemoleküls konjugiert sind.

4. Therapeutisch wirksame Menge einer Kombination aus zwei oder mehreren kurzkettigen Fettsäuren, Estern oder Salzen davon zur Verwendung nach Anspruch 3, wobei bei dem Individuum ein Risiko eines Entwickelns einer Autoimmunerkrankung bestimmt wird, vorzugsweise wobei bei dem Individuum bestimmt wird, Autoantikörper oder Entzündungsmarker aufzuweisen, die mit einem Risiko des Entwickelns einer Autoimmunerkrankung verknüpft sind.

5. Therapeutisch wirksame Menge einer Kombination aus zwei oder mehreren kurzkettigen Fettsäuren, Estern oder Salzen davon zur Verwendung nach einem der vorstehenden Ansprüche, wobei die Autoimmunerkrankung aus der Gruppe ausgewählt ist, bestehend aus: Typ-1-Diabetes mellitus, Psoriasis, rheumatoider Arthritis, Reizdarmsysdrom, Zöliakie, Autoimmunhepatitis, Myokarditis, Lupusnephritis, Multipler Sklerose oder primärer biliärer Zirrhose, vorzugsweise wobei die Autoimmunerkrankung um Typ-1-Diabetes mellitus ist.

6. Therapeutisch wirksame Menge einer Kombination aus zwei oder mehreren kurzkettigen Fettsäuren, Estern oder Salzen davon zur Verwendung nach einem der vorstehenden Ansprüche, wobei die Autoimmunerkrankung Typ-1-Diabetes mellitus oder Psoriasis ist.

7. Therapeutisch wirksame Menge einer Kombination aus kurzkettigen Fettsäuren, Estern oder Salzen davon zur Verwendung nach einem der vorstehenden Ansprüche, wobei die kurzkettigen Fettsäuren in Form einer Kombination aus Stärkemolekülen, die an Essigsäure kovalent gebunden sind, und aus Stärkemolekülen vorliegen, die an Buttersäure kovalent gebunden sind.

8. Therapeutisch wirksame Menge einer Kombination aus kurzkettigen Fettsäuren, Estern oder Salzen davon zur Verwendung nach einem der Ansprüche 1 bis 6, wobei jedes Stärkemolekül mit mindestens einem Essigsäuremolekül und mindestens einem Buttersäuremolekül konjugiert ist.

9. Diätetisches Mittel für eine Verabreichung einer Kombination kurzkettiger Fettsäuren in den Dickdarm eines_Individuums, wobei das Mittel eine Stärke einschließt, die durch eine Bindung, die in das Kolon eines Individuums hydrolysierbar ist, an die kurzkettigen Fettsäuren kovalent gebunden ist, um freie Fettsäure zu geben, wobei die Kombination kurzkettiger Fettsäuren Buttersäure und Essigsäure ist, vorzugsweise wobei die Kombination ferner Propionsäure einschließt.

10. Diätetisches Mittel nach Anspruch 9, wobei das diätetische Mittel eine Kombination aus Stärkemolekülen, die an Essigsäure kovalent gebunden sind, und aus Stärkemolekülen umfasst, die an Buttersäure kovalent gebunden sind.

11. Diätetisches Mittel nach Anspruch 9, wobei das diätetische Mittel Stärkemoleküle umfasst, die an mindestens ein Buttersäuremolekül und mindestens ein Essigsäuremolekül kovalent gebunden sind.

12. Pharmazeutische Zusammensetzung zur Verwendung bei dem Behandeln oder zur Verwendung bei dem Verzögern des Fortschreitens einer Autoimmunerkrankung, wobei die Zusammensetzung eine Kombination aus Buttersäure und Essigsäure, Estern oder Salzen davon und pharmazeutisch verträglichen Arzneistoffträgern einschließt, wobei die Buttersäure, Essigsäure, Ester oder Salze davon die aktiven Bestandteile in der Zusammensetzung sind und wobei die Buttersäure und Essigsäure an eine Trägersubstanz in Form eines Stärkemoleküls konjugiert sind.

13. Pharmazeutische Zusammensetzung zur Verwendung nach Anspruch 12, wobei die Zusammensetzung eine Kombination von Stärkemolekülen, die an Essigsäure kovalent gebunden sind, und von Stärkemolekülen umfasst, die an Buttersäure kovalent gebunden sind.

14. Pharmazeutische Zusammensetzung zur Verwendung nach Anspruch 12, wobei die Zusammensetzung ein Stärkemolekül umfasst, das mit mindestens einem Essigsäuremolekül und mindestens einem Buttersäuremolekül konjugiert ist.

15. Diätetisches Mittel zur Verwendung nach einem der Ansprüche 9 bis 11 oder pharmazeutische Zusammensetzung zur Verwendung nach den Ansprüchen 12 bis 14, wobei das Mittel oder die Zusammensetzung zum Behandeln oder Verzögern des Fortschreitens von Typ-1-Diabetes mellitus oder Psoriasis dient.

## Revendications

1. Quantité thérapeutiquement efficace d'une combinaison d'au moins deux acides gras à chaîne courte, de leurs esters ou de leurs sels, à utiliser pour traiter une maladie auto-immune ou pour retarder ou prévenir la progression d'une maladie auto-immune chez un individu,
dans laquelle la combinaison d'acides gras à chaîne courte est l'acide butyrique et l'acide acétique, de préférence dans laquelle la combinaison comporte en outre l'acide propionique, et dans laquelle les acides gras à chaîne courte sont conjugués à un support sous la forme d'une molécule d'amidon.

2. Quantité thérapeutiquement efficace d'une combinaison d'au moins deux acides gras à chaîne courte, de leurs esters ou de leurs sels pour l'utilisation de la revendication 1, dans laquelle le traitement de la maladie auto-immune comporte la réduction ou le traitement de l'inflammation chez l'individu, de préférence dans laquelle la réduction ou le traitement de l'inflammation comporte la réduction de la proportion d'une ou de plusieurs cytokines pro-inflammatoires chez l'individu ou l'augmentation de la proportion d'une ou de plusieurs cytokines anti-inflammatoires chez l'individu.

3. Quantité thérapeutiquement efficace d'une combinaison d'au moins deux acides gras à chaîne courte, d'esters ou de sels de ceux-ci, à utiliser pour prévenir ou retarder l'apparition d'une maladie auto-immune chez un individu,
dans laquelle la combinaison d'acides gras à chaîne courte, d'esters ou de sels de ceux-ci, est l'acide butyrique et l'acide acétique ou des esters ou des sels de ceux-ci, de préférence dans laquelle la combinaison comporte en outre l'acide propionique, et dans laquelle les acides gras à chaîne courte sont conjugués à un support sous la forme d'une molécule d'amidon.

4. Quantité thérapeutiquement efficace d'une combinaison d'au moins deux acides gras à chaîne courte, de leurs esters ou de leurs sels pour l'utilisation selon la revendication 3, dans laquelle il est déterminé que l'individu est à risque de développer une maladie auto-immune, de préférence dans laquelle il est déterminé que l'individu a des auto-anticorps ou des marqueurs inflammatoires associés à un risque de développement d'une maladie auto-immune.

5. Quantité thérapeutiquement efficace d'une combinaison d'au moins deux acides gras à chaîne courte, de leurs esters ou de leurs sels pour l'utilisation selon l'une quelconque des revendications précédentes, dans laquelle la maladie autoimmune est choisie dans le groupe constitué par : le diabète de type 1, le psoriasis, la polyarthrite rhumatoïde, les maladies inflammatoires de l'intestin, la maladie cœliaque, l'hépatite auto-immune, la myocardite, le lupus néphrétique, la sclérose en plaques ou la cirrhose biliaire primitive, de préférence dans laquelle la maladie auto-immune est le diabète de type 1.

6. Quantité thérapeutiquement efficace d'une combinaison d'au moins deux acides gras à chaîne courte, de leurs esters ou de leurs sels pour l'utilisation selon l'une quelconque des revendications précédentes, dans laquelle la maladie autoimmune est le diabète de type 1 ou le psoriasis.

7. Quantité thérapeutiquement efficace d'une combinaison d'acides gras à chaîne courte, de leurs esters ou de leurs sels, pour l'utilisation selon l'une quelconque des revendications précédentes, dans laquelle les acides gras à chaîne courte se présentent sous la forme d'une combinaison de molécules d'amidon liées de manière covalente à l'acide acétique et de molécules d'amidon liées de manière covalente à l'acide butyrique.

8. Quantité thérapeutiquement efficace d'une combinaison d'acides gras à chaîne courte, de leurs esters ou de leurs sels, pour l'utilisation selon l'une quelconque des revendications 1 à 6, dans laquelle chaque molécule d'amidon est conjuguée à au moins une molécule d'acide acétique et à au moins une molécule d'acide butyrique.

9. Agent diététique pour l'administration d'une combinaison d'acides gras à chaîne courte dans le gros intestin d'un individu, l'agent comportant un amidon lié de manière covalente aux acides gras à chaîne courte par une liaison qui est hydrolysable dans le côlon d'un individu, pour donner un acide gras libre, dans lequel la combinaison d'acides gras à chaîne courte est l'acide butyrique et l'acide acétique, de préférence dans lequel la combinaison comprend en outre l'acide propionique.

10. Agent diététique selon la revendication 9, dans lequel l'agent diététique comprend une combinaison de molécules d'amidon liées de manière covalente à l'acide acétique et de molécules d'amidon liées de manière covalente à l'acide butyrique.

11. Agent diététique selon la revendication 9, dans lequel l'agent diététique comprend des molécules d'amidon liées de manière covalente à au moins un acide butyrique et à au moins une molécule acétique.

12. Composition pharmaceutique utilisée pour traiter ou retarder la progression d'une maladie auto-immune, dans laquelle la composition comprend une combinaison d'acide butyrique et d'acide acétique, d'esters ou de sels de ceux-ci et d'excipients pharmaceutiquement acceptables, dans laquelle l'acide butyrique, l'acide acétique, les esters ou les sels de ceux-ci sont les ingrédients actifs de la composition, et dans laquelle l'acide butyrique et l'acide acétique sont conjugués à un transporteur sous la forme d'une molécule d'amidon.

13. Composition pharmaceutique selon la revendication 12, dans laquelle la composition comprend une combinaison de molécules d'amidon liées de manière covalente à l'acide acétique et de molécules d'amidon liées de manière covalente à l'acide butyrique

14. Composition pharmaceutique selon la revendication 12, dans laquelle la composition comprend une molécule d'amidon conjuguée à au moins une molécule d'acide acétique et à au moins une molécule d'acide butyrique.

15. Agent diététique utilisé selon l'une quelconque des revendications 9 à 11, ou composition pharmaceutique utilisée selon l'une quelconque des revendications 12 à 14, dans lequel l'agent ou la composition sont destinés à traiter ou à retarder la progression du diabète de type 1 ou du psoriasis.
